(19) Europäisches Patentamt
European Patent Office
Office européen des brevets

(11) EP 2 353 584 B1

(12) # FASCICULE DE BREVET EUROPEEN

(45) Date de publication et mention de la délivrance du brevet:
**23.08.2017 Bulletin 2017/34**

(21) Numéro de dépôt: **10163820.3**

(22) Date de dépôt: **25.05.2010**

(51) Int Cl.:
***A61K 8/81*** *(2006.01)*
***A61K 8/90*** *(2006.01)*
***A61Q 1/06*** *(2006.01)*
***A61K 8/37*** *(2006.01)*
***A61Q 1/04*** *(2006.01)*

(54) **Composition cosmétique comprenant un polymère séquencé et une huile ester non volatile**

kosmetische Zusammensetzung enthaltend ein Block-Polymer und eine nicht-flüchtiges Esteröl

Cosmetic composition based on a block polymer and a non volatile ester oil

(84) Etats contractants désignés:
**AL AT BE BG CH CY CZ DE DK EE ES FI FR GB GR HR HU IE IS IT LI LT LU LV MC MK MT NL NO PL PT RO SE SI SK SM TR**

(30) Priorité: **19.06.2009 FR 0954170**
**02.06.2009 FR 0953625**
**01.06.2009 FR 0902617**

(43) Date de publication de la demande:
**10.08.2011 Bulletin 2011/32**

(73) Titulaire: **L'Oréal**
**75008 Paris (FR)**

(72) Inventeurs:
- **Ilekti, Philippe**
**94700, Maison-Alfort (FR)**
- **Farcet, Céline**
**93320, Les Pavillons sous Bois (FR)**
- **Schvent, Alexandra**
**94160, Saint Mande (FR)**

(74) Mandataire: **Boulard, Denis**
**L'Oréal**
**Service DIPI**
**9 Rue Pierre Dreyfus**
**92110 Clichy (FR)**

(56) Documents cités:
**EP-A1- 1 184 028**
**EP-A1- 1 882 709**
**EP-A1- 1 884 229**
**EP-A2- 1 411 069**
**WO-A2-2004/028487**
**WO-A2-2004/028488**
**FR-A1- 2 860 155**
**FR-A1- 2 860 156**
**US-A1- 2008 171 005**
**US-A1- 2008 171 006**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

EP 2 353 584 B1

## Description

La présente invention a pour objet une composition cosmétique pour le maquillage et/ou le soin des matières kératiniques, en particulier des lèvres et de la peau.

Dans le cadre de la présente invention, le terme « matières kératiniques » comprend la peau, les lèvres, les ongles, les cheveux, les cils et les sourcils.

De telles compositions de maquillage et/ou de soin de la peau et/ou des lèvres contiennent classiquement un polymère filmogène connu pour améliorer la tenue de ces compositions sur les matières kératiniques, et notamment, lorsqu'il s'agit d'un rouge à lèvres, la tenue dudit rouge à lèvres sur les lèvres.

Toutefois, de tels polymères filmogènes sont généralement véhiculés dans des huiles volatiles (celles-ci pouvant par exemple être utilisées en tant que solvant de polymérisation du polymère filmogène) qui peuvent présenter des sensations d'inconfort à l'application et nuire à la brillance du dépôt de rouges à lèvres. De plus, la présence de cette huile volatile implique des contraintes en termes de procédé de préparation en particulier pour la préparation de rouges à lèvres comprenant des corps gras solides tels que des cires ayant un point de fusion élevé, pour lesquels il est nécessaire de chauffer à une température généralement supérieure à celle du point éclair de l'huile volatile.

Par ailleurs, ces compositions contenant une huile volatile doivent être conditionnées dans un packaging (emballage) afin de préserver toute évaporation du solvant (c'est-à-dire de l'huile volatile) de la composition pendant le stockage. Cette contrainte du packaging représente un coût supplémentaire.

Il est connu des documents EP 1 411 069, EP 1 882 709 et EP 1 884 229, des compositions cosmétiques contenant des polymères séquencés véhiculés dans une huile volatile dont le point éclair est inférieur à 80°C (comme par exemple l'isododécane).

Ces polymères séquencés sont notamment synthétisés en présence d'isododécane. Or, le polymère séquencé, véhiculé dans une huile volatile dont le point éclair est inférieur à 80°C, telle que l'isododécane, ne peut être formulé qu'avec des additifs cosmétiques ne nécessitant pas d'étape de chauffage à une température supérieure à celle du point éclair de ladite huile volatile.

Ainsi, par exemple, l'introduction d'un corps gras solide, tel qu'une cire ou un corps gras pâteux, présentant un point de fusion supérieur audit point éclair de ladite huile volatile ne pourra pas être réalisée ; en effet il serait nécessaire de chauffer le mélange polymère, huile volatile, corps gras solide à une température supérieure au point éclair dudit solvant volatil.

En outre, la présence d'une quantité élevée d'huile volatile dont le point éclair est inférieur à 80°C nuit à l'obtention de bonnes propriétés cosmétiques d'une composition cosmétique à appliquer sur les lèvres ou la peau. En effet, une teneur élevée (c'est-à-dire supérieure à 10%) en huile volatile engendre une sensation de sécheresse et de tiraillement sur les lèvres ou la peau (sensation d'inconfort), ainsi qu'une diminution de la brillance du dépôt formé après application de la composition sur les lèvres ou la peau.

Il existe donc un besoin de disposer d'une composition cosmétique contenant un polymère filmogène séquencé permettant d'obtenir un dépôt sur les lèvres ou la peau présentant de bonnes propriétés de brillance et de confort.

Les inventeurs ont découvert qu'une telle composition pouvait être obtenue en associant le polymère filmogène séquencé à des huiles esters non volatiles particulières.

Une telle composition appliquée sur les matières kératiniques, notamment la peau ou les lèvres, permet d'obtenir un dépôt (notamment de maquillage) présentant de bonnes propriétés de confort (absence de sensation de tiraillement, de sècheresse) et de brillance.

Cette composition permet également d'obtenir un maquillage des matières kératiniques (peau et lèvres notamment) présentant une bonne tenue de la couleur (pendant au moins 6h).

Il est connu dans la demande EP 1 184 028 de formuler des compositions cosmétiques comprenant au moins un composé siliconé non volatil, au moins une huile hydrocarbonée non volatile et une phase particulaire inerte afin de fournir des compositions brillantes et sans transfert. Cependant, ce document ne met pas en oeuvre un polymère filmogène tel que dans la présente invention.

Ainsi, selon un premier aspect, la présente invention a pour objet une composition cosmétique pour le maquillage et/ou le soin des matières kératiniques, et notamment des lèvres ou de la peau, comprenant, dans un milieu physiologiquement acceptable, au moins :

a) un copolymère éthylénique séquencé, contenant au moins une première séquence ayant une température de transition vitreuse (Tg) supérieure ou égale à 40°C et étant issue en totalité ou en partie de un ou plusieurs premiers monomères, qui sont tels que l'homopolymère préparé à partir de ces monomères a une température de transition vitreuse supérieure ou égale à 40°C, ladite première séquence étant obtenue à partir :

- d'au moins un monomère méthacrylate de formule $CH_2 = C(CH_3)\text{-}COOR'_2$ dans laquelle $R'_2$ représente un groupe cycloalkyle $C_4$ à $C_{12}$, et

- d'au moins un monomère acrylate de formule $CH_2 = CH\text{-}COOR_2$

dans laquelle $R_2$ représente un groupe cycloalkyle en $C_4$ à $C_{12}$,
et au moins une deuxième séquence ayant une température de transition vitreuse inférieure ou égale à 20°C et étant issue en totalité ou en partie de un ou plusieurs deuxièmes monomères, qui sont tels que l'homopolymère préparé à partir de ces monomères a une température de transition vitreuse inférieure ou égale à 20°C, et d'un monomère additionnel choisi parmi l'acide acrylique ;
ladite première séquence et ladite deuxième séquence étant reliées entre elles par un segment intermédiaire statistique comprenant au moins un desdits premiers monomères constitutifs de la première séquence et au moins un desdits deuxièmes monomères constitutifs de la deuxième séquence, et ledit copolymère séquencé ayant un indice de polydispersité I supérieur à 2, et
b) une huile ester hydrocarbonée non volatile comprenant au moins 16 atomes de carbone et ayant une masse molaire inférieure à 650 g/mole ;

ladite composition comprenant moins de 10% en poids d'huiles volatiles dont le point éclair est inférieur ou égal à 80°C (telles que l'isododécane), ou mieux moins de 5 % par rapport au poids total de la composition ou encore étant exempte d'huiles volatiles dont le point éclair est inférieur ou égal à 80°C.

L'invention a également pour objet, selon un autre aspect, un procédé cosmétique de maquillage des matières kératiniques comprenant l'application sur lesdites matières kératiniques, et notamment les lèvres d'une composition telle que définie précédemment.

L'invention a enfin pour objet l'utilisation d'un copolymère séquencé tel que décrit précédemment, associé à au moins une huile ester hydrocarbonée non volatile comprenant au moins 16 atomes de carbone et ayant une masse molaire inférieure à 650 g/mole, dans une composition comprenant moins de 10%, ou mieux moins de 5 %, ou encore étant exempte d'huiles volatiles dont le point éclair est inférieur ou égal à 80°C (telles que l'isododécane), la composition étant destinée à procurer un dépôt sur les matières kératiniques, en particulier les lèvres, présentant des propriétés de confort, brillance, et avantageusement de tenue de la brillance, dotée d'une longue tenue.

L'utilisation de cette association a pour avantage de procurer un film d'une cosméticité accrue.

Dans le cadre de la présente invention, le terme « matières kératiniques » comprend la peau, les lèvres, les ongles, les cheveux, les cils et les sourcils.

Selon un premier mode de réalisation, la composition selon l'invention est liquide.

Selon un second mode de réalisation, la composition selon l'invention est solide.

Les termes « solide » et « liquide » caractérisent l'état de la composition à température ambiante et à pression atmosphérique (760 mm de Hg).

**Protocole de mesure de la dureté :**

La mesure est réalisée selon le protocole suivant :

Le stick de rouge à lèvres est conservé à 20 °C pendant 24 heures avant la mesure de la dureté.

La dureté peut être mesurée à 20 °C par la méthode dite « du fil à couper le beurre », qui consiste à couper transversalement un bâton de produit, de préférence cylindrique de révolution, à l'aide d'un fil rigide de tungstène de diamètre 250 µm en déplaçant le fil relativement au stick à une vitesse de 100 mm/min.

La dureté des échantillons de compositions de l'invention, exprimée en $Nm^{-1}$, est mesurée au moyen d'un dynamomètre DFGS2 commercialisé par la société INDELCO-CHATILLON.

La mesure est reproduite trois fois puis moyennée. La moyenne des trois valeurs lues au moyen du dynamomètre mentionné ci-dessus, notée Y, est donnée en grammes. Cette moyenne est convertie en Newton puis divisée par L qui représente la dimension la plus élevée traversée par le fil. Dans le cas d'un bâton cylindrique, L est égal au diamètre (en mètres).

La dureté est convertie en $Nm^{-1}$ par l'équation ci-dessous :

$$(Y x\, 10^{-3}\, x 9.8)/L$$

Pour une mesure à une température différente, on conserve le stick 24 heures à cette nouvelle température avant la

mesure.

Selon cette méthode, la dureté à 20 °C d'exemples de composition selon un aspect de l'invention est supérieure à 30 $Nm^{-1}$ notamment supérieure à 40 $Nm^{-1}$de préférence supérieure à 50 $Nm^{-1}$.

Selon cette méthode, la dureté à 20 °C d'exemples de composition selon un aspect de l'invention est inférieure à 500 $Nm^{-1}$ notamment inférieure à 400 $Nm^{-1}$ de préférence inférieure à 300 $Nm^{-1}$.

En particulier, par « composition solide » on entend une composition dont la dureté est supérieure à 30 $Nm^{-1}$.

## COPOLYMERE ETHYLENIQUE SEQUENCE

La composition selon la présente invention contient au moins un copolymère éthylénique séquencé (également appelé polymère éthylénique séquencé), contenant au moins une première séquence ayant une température de transition vitreuse (Tg) supérieure ou égale à 40°C et étant issue en totalité ou en partie de plusieurs premiers monomères, qui seront définis plus loin, et qui sont tels que l'homopolymère préparé à partir de ces monomères a une température de transition vitreuse supérieure ou égale à 40°C, et au moins une deuxième séquence ayant une température de transition vitreuse inférieure ou égale à 20°C et étant issue en totalité ou en partie de un ou plusieurs deuxièmes monomères, qui sont tels que l'homopolymère préparé à partir de ces monomères a une température de transition vitreuse inférieure ou égale à 20°C, et d'un monomère additionnel choisi parmi l'acide acrylique ; ladite première séquence et ladite deuxième séquence étant reliées entre elles par un segment intermédiaire statistique comprenant au moins un desdits premiers monomères constitutifs de la première séquence et au moins un desdits deuxièmes monomères constitutifs de la deuxième séquence, et ledit copolymère séquencé ayant un indice de polydispersité I supérieur à 2.

Le polymère séquencé utilisé selon l'invention comprend ainsi au moins une première séquence et au moins une deuxième séquence.

Par "au moins" une séquence, on entend une ou plusieurs séquences.

Par polymère "séquencé", on entend un polymère comprenant au moins 2 séquences distinctes, de préférence au moins 3 séquences distinctes.

Par polymère "éthylénique", on entend un polymère obtenu par polymérisation de monomères comprenant une insaturation éthylénique.

Le polymère éthylénique séquencé utilisé selon l'invention est préparé exclusivement à partir de monomères monofonctionnels.

Cela signifie que le polymère éthylénique séquencé utilisé selon la présente invention ne contient pas de monomères multifonctionnels, qui permettent de casser la linéarité d'un polymère afin d'obtenir un polymère branché ou voire réticulé, en fonction du taux de monomère multifonctionnel. Le polymère utilisé selon l'invention ne contient pas non plus de macromonomères (par « macromonomère » on entend un monomère monofonctionnel ayant un groupe pendant de nature polymérique, et ayant de préférence une masse moléculaire supérieure à 500 g/mol, ou bien un polymère comportant sur une seule de ses extrémités un groupe terminal polymérisable (ou à insaturation éthylénique)), qui sont utilisés à la préparation d'un polymère greffé.

On précise que dans ce qui précède et ce qui suit les termes "première" et "deuxième" séquences ne conditionnent nullement l'ordre desdites séquences (ou blocs) dans la structure du polymère.

La première séquence et la deuxième séquence du polymère utilisé dans l'invention peuvent être avantageusement incompatibles l'une avec l'autre.

Par "séquences incompatibles l'une avec l'autre", on entend que le mélange formé par un polymère correspondant à la première séquence et par un polymère correspondant à la deuxième séquence, n'est pas miscible dans le solvant de polymérisation, majoritaire en poids, du polymère séquencé, à température ambiante (25°C) et pression atmosphérique ($10^5$ Pa), pour une teneur du mélange desdits polymères supérieure ou égale à 5 % eh poids, par rapport au poids total du mélange desdits polymères et dudit solvant de polymérisation, étant entendu que :

i) lesdits polymères sont présents dans le mélange en une teneur telle que le rapport pondéral respectif va de 10/90 à 90/10, et que

ii) chacun des polymères correspondant au première et seconde séquences a une masse moléculaire moyenne (en poids ou en nombre) égale à celle du polymère séquence +/- 15%.

Dans le cas d'un mélange de solvants de polymérisation, dans l'hypothèse de deux ou plusieurs solvants présents en proportions massiques identiques, ledit mélange de polymères est non miscible dans au moins l'un d'entre eux.

Bien entendu, dans le cas d'une polymérisation réalisée dans un solvant unique, ce dernier est le solvant majoritaire.

Le polymère séquence selon l'invention comprend au moins une première séquence et au moins une deuxième séquence reliées entre elles par un segment intermédiaire comprenant au moins un monomère constitutif de la première

séquence et au moins un monomère constitutif de la deuxième séquence. Le segment intermédiaire (également appelé séquence intermédiaire) a une température de transition vitreuse Tg comprise entre les températures de transition vitreuse des première et deuxième séquences.

Le segment intermédiaire est une séquence comprenant au moins un monomère constitutif de la première séquence et au moins un monomère constitutif de la deuxième séquence du polymère permet de "compatibiliser" ces séquences.

Avantageusement, le segment intermédiaire comprenant au moins un monomère constitutif de la première séquence et au moins un monomère constitutif de la deuxième séquence du polymère est un polymère statistique.

De préférence, la séquence intermédiaire est issue essentiellement de monomères constitutifs de la première séquence et de la deuxième séquence.

Par "essentiellement", on entend au moins à 85%, de préférence au moins à 90%, mieux à 95% et encore mieux à 100%.

Le polymère séquencé selon l'invention est avantageusement un polymère éthylénique séquencé filmogène.

Par polymère "éthylénique", on entend un polymère obtenu par polymérisation de monomères comprenant une insaturation éthylénique.

Par polymère "filmogène", on entend un polymère apte à former à lui seul ou en présence d'un agent auxiliaire de filmification, un dépôt continu sur un support, notamment sur les matières kératiniques.

De façon préférentielle, le polymère selon l'invention ne comprend pas d'atomes de silicium dans son squelette. Par "squelette", on entend la chaîne principale du polymère, par opposition aux chaînes latérales pendantes.

De préférence, le polymère selon l'invention n'est pas hydrosoluble, c'est à dire que le polymère n'est pas soluble dans l'eau ou dans un mélange d'eau et de monoalcools inférieurs linéaires ou ramifiés ayant de 2 à 5 atomes de carbone comme l'éthanol, l'isopropanol ou le n-propanol, sans modification de pH, à une teneur en matière active d'au moins 1 % en poids, à température ambiante (25°C).

De préférence, le polymère selon l'invention n'est pas un élastomère.

Par "polymère non élastomère", on entend un polymère qui, lorsqu'il est soumis à une contrainte visant à l'étirer (par exemple de 30% relativement à sa longueur initiale), ne revient pas à une longueur sensiblement identique à sa longueur initiale lorsque cesse la contrainte.

De manière plus spécifique, par "polymère non élastomére" on désigne un polymère ayant une recouvrance instantanée $R_i$ < à 50% et une recouvrance retardée $R_{2h}$ < 70% après avoir subi un allongement de 30%. De préférence, $R_i$ est < à 30 %, et $R_{2h}$ < 50%.

Plus précisément, le caractère non élastomérique du polymère est déterminé selon le protocole suivant :

> On prépare un film de polymère par coulage d'une solution du polymère dans une matrice téflonnée puis séchage pendant 7 jours dans une ambiance contrôlée à 23±5°C, et 50±10 % d'humidité relative.

On obtient alors un film d'environ 100 μm d'épaisseur dans lequel sont découpées des éprouvettes rectangulaires (par exemple à l'emporte-pièce) d'une largeur de 15 mm et d'une longueur de 80 mm.

On impose à cet échantillon une sollicitation de traction à l'aide d'un appareil commercialisé sous la référence Zwick, dans les mêmes conditions de température et d'humidité que pour le séchage.

Les éprouvettes sont étirées à une vitesse de 50 min/min et la distance entre les mors est de 50 mm, ce qui correspond à la longueur initiale ($I_0$) de l'éprouvette.

On détermine la recouvrance instantanée Ri de la manière suivante :

- on étire l'éprouvette de 30 % ($\varepsilon_{max}$) c'est-à-dire environ 0,3 fois sa longueur initiale ($I_0$)
- on relâche la contrainte en imposant une vitesse de retour égale à la vitesse de traction, soit 50 mm/min et on mesure l'allongement résiduel de l'éprouvette en pourcentage, après retour à contrainte charge nulle ($\varepsilon_i$).

La recouvrance instantanée en % ($R_i$) est donnée par la formule ci-après:

$$R_i = (\varepsilon_{max} - \varepsilon_i)/ \varepsilon_{max}) \times 100$$

Pour déterminer la recouvrance retardée, on mesure après 2 heures le taux d'allongement résiduel de l'éprouvette en pourcentage ($\varepsilon_{2h}$), 2 heures après retour à la contrainte charge nulle.

La recouvrance retardée en % ($R_{2h}$) est donnée par la formule ci-après:

$$R_{2h} = (\varepsilon_{max} - \varepsilon_{2h}) / \varepsilon_{max}) \times 100$$

A titre purement indicatif, un polymère selon un mode de réalisation de l'invention possède de préférence une recouvrance instantanée $R_i$ de 10% et une recouvrance retardée $R_{2h}$ de 30%.

L'indice de polydispersité du polymère de l'invention est supérieur à 2.

Avantageusement, le polymère séquencé utilisé dans les compositions selon l'invention a un indice de polydispersité 1 supérieur à 2, par exemple allant de 2 à 9, de préférence supérieur ou égal à 2,5, par exemple allant de 2,5 à 8, et mieux supérieur ou égal à 2,8 et notamment, allant de 2,8 à 6.

L'indice de polydispersité I du polymère est égal au rapport de la masse moyenne en poids Mw sur la masse moyenne en nombre Mn.

On détermine les masses molaires moyennes en poids (Mw) et en nombre (Mn) par chromatographie liquide par perméation de gel (solvant THF, courbe d'étalonnage établie avec des étalons de polystyrène linéaire, détecteur réfractométrique).

La masse moyenne en poids (Mw) du polymère selon l'invention est de préférence inférieure ou égale à 300 000, elle va par exemple de 35 000 à 200 000, et mieux de 45 000 à 150 000 g/mol.

La masse moyenne en nombre (Mn) du polymère selon l'invention est de préférence inférieure ou égale à 70 000, elle va par exemple de 10 000 à 60 000, et mieux de 12 000 à 50 000 g/mol.

De préférence, l'indice de polydispersité du polymère selon l'invention est supérieur à 2, par exemple allant de 2 à 9, de préférence supérieur ou égal à 2,5, par exemple allant de 2,5 à 8, et mieux supérieur ou égal à 2,8 et notamment, allant de 2,8 à 6.

**Première séquence ayant une Tg supérieure ou égale à 40°C**

La séquence ayant une Tg supérieure ou égale à 40°C a par exemple une Tg allant de 40 à 150°C, de préférence supérieure ou égale à 50°C, allant par exemple de 50°C à 120 °C, et mieux supérieure ou égale à 60°C, allant par exemple de 60°C à 120°C.

Les températures de transition vitreuse indiquées des première et deuxième séquences peuvent être des Tg théoriques déterminées à partir des Tg théoriques des monomères constitutifs de chacune des séquences, que l'on peut trouver dans un manuel de référence tel que le Polymer Handbook, 3rd ed, 1989, John Wiley, selon la relation suivante, dite Loi de Fox :

$$1/Tg = \sum_i (\varpi_i / Tg_i) ,$$

$\overline{\omega}_i$ étant la fraction massique du monomère i dans la séquence considérée et $Tg_i$ étant la température de transition vitreuse de l'homopolymère du monomère i.

Sauf indication contraire, les Tg indiquées pour les première et deuxième séquences dans la présente demande sont des Tg théoriques.

L'écart entre les températures de transition vitreuse des première et deuxième séquences est généralement supérieur à 10°C, de préférence supérieur à 20°C, et mieux supérieur à 30°C.

On entend désigner dans la présente invention, par l'expression « compris entre ... et ... », un intervalle de valeurs dont les bornes mentionnées sont exclues, et « de ... à ... » et « allant de ... à ... », un intervalle de valeurs dont les bornes sont inclues.

La séquence ayant une Tg supérieure ou égale à 40°C peut être un homopolymère ou un copolymère.

La séquence ayant une Tg supérieure ou égale à 40°C peut être issue en totalité ou en partie de un ou plusieurs monomères, qui sont tels que l'homopolymère préparé à partir de ces monomères a une température de transition vitreuse supérieure ou égale à 40°C. Cette séquence peut également être appelée « séquence rigide ».

Selon l'invention, la première séquence est un copolymère, elle est issue en totalité ou en partie de de plusieurs monomères, dont la nature et la concentration sont choisies de façon que la Tg du copolymère résultant soit supérieure ou égale à 40°C. Le copolymère peut par exemple comprendre :

- des monomères qui sont tel(s) que les homopolymères préparés à partir de ces monomères ont des Tg supérieures ou égales à 40°C, par exemple une Tg allant de 40°C à 150 °C, de préférence supérieure ou égale à 50°C, allant par exemple de 50°C à 120°C, et mieux supérieure ou égale à 60°C, allant par exemple de 60°C à 120°C, et
- des monomères qui sont tel(s) que les homopolymères préparés à partir de ces monomères ont des Tg inférieures

à 40°C, choisis parmi les monomères ayant une Tg comprise entre 20°C à 40°C et/ou les monomères ayant une Tg inférieure ou égale à 20°C, par exemple une Tg allant de -100°C à 20°C, de préférence inférieure à 15°C, notamment allant de - 80°C à 15°C et mieux inférieur à 10°C, par exemple allant de -50°C à 0°C à, tels que décrits plus loin.

La première séquence est obtenue à partir d'au moins un monomère acrylate de formule $CH_2$ = CH-$COOR_2$ et d'au moins un monomère méthacrylate de formule $CH_2$ = C($CH_3$)-$COOR_2$ dans laquelle $R_2$ représente un groupe cycloalkyle $C_4$ à $C_{12}$, de préférence un cycloalkyle $C_8$ à $C_{12}$, tel que l'isobornyle. Les monomères et leurs proportions sont de préférence choisis de telle sorte que la température de transition vitreuse de la première séquence est supérieure ou égale à 40°C.

Selon l'invention, la première séquence est obtenue à partir :

i) d'au moins un monomère acrylate de formule $CH_2$ = CH-$COOR_2$ dans laquelle $R_2$ représente un groupe cycloalkyle $C_4$ à $C_{12}$, de préférence un groupe cycloalkyle en $C_8$ à $C_{12}$, tel que l'isobornyle,

- ii) et d'au moins un monomère méthacrylate de formule $CH_2$ = C($CH_3$)-COOR'$_2$ dans laquelle R'$_2$ représente un groupe cycloalkyle $C_4$ à $C_{12}$, de préférence un groupe cycloalkyle en $C_8$ à $C_{12}$, tel que l'isobornyle.

Selon un mode de mise en oeuvre, la première séquence est obtenue à partir d'au moins un monomère acrylate de formule $CH_2$ = CH-$COOR_2$ dans laquelle $R_2$ représente un groupe cycloalkyle $C_8$ à $C_{12}$, tel que l'isobornyle, et d'au moins un monomère méthacrylate de formule $CH_2$ = C($CH_3$)-COOR'$_2$ dans laquelle R'$_2$ représente un groupe cycloalkyle $C_8$ à $C_{12}$,, tel que l'isobornyle.

De façon préférée, $R_2$ et R'$_2$ représentent indépendamment ou simultanément un groupe isobornyle.

De façon préférée, le copolymère séquencé comprend de 50 à 80 % en poids de méthacrylate/acrylate d'isobornyle, de 10 à 30 % en poids d'acrylate d'isobutyle et de 2 à 10 % en poids d'acide acrylique.

La première séquence peut être obtenue exclusivement à partir dudit monomère acrylate et dudit monomère méthacrylate.

Le monomère acrylate et le monomère méthacrylate sont de préférence dans des propositions massiques comprises entre 30 :70 et 70 :30, de préférence entre 40 :60 et 60 :40, notamment de l'ordre de 50 :50.

La proportion de la première séquence va avantageusement de 20 à 90% en poids du polymère, mieux de 30 à 80% et encore mieux de 60 à 80%.

Selon un mode de mise en oeuvre, la première séquence est obtenue par polymérisation du méthacrylate d'isobornyle et de l'acrylate d'isobornyle:

**Deuxième séquence de température de transition vitreuse inférieure à 20°C.**

La deuxième séquence a avantageusement une température de transition vitreuse Tg inférieure ou égale à 20°C a par exemple une Tg allant de -100°C à 20°C, de préférence inférieure ou égale à 15°C, notamment allant de -80°C à 15°C et mieux inférieure ou égale à 10°C, par exemple allant de - 100 °C à 10 °C, notamment allant de - 30°C à 10°C.

La deuxième séquence est issue en totalité ou en partie de un ou plusieurs deuxièmes monomères, qui sont tels que l'homopolymère préparé à partir de ces monomères a une température de transition vitreuse inférieure ou égale à 20°C.

Cette séquence peut également être appelée « séquence souple ».

Le monomère ayant une Tg inférieure ou égale à 20°C (appelé deuxième monomère) est, de préférence, choisi parmi les monomères suivants:

- les acrylates de formule $CH_2$ = $CHCOOR_3$,
  $R_3$ représentant un groupe alkyle non substitué en $C_1$ à $C_{12}$, linéaire ou ramifié, à l'exception du groupe tertiobutyle, dans lequel se trouve(nt) éventuellement intercalé(s) un ou plusieurs hétéroatomes choisis parmi O, N, S,
- les méthacrylates de formule $CH_2$ = C($CH_3$)-$COOR_4$,
  $R_4$ représentant un groupe alkyle non substitué en $C_6$ à $C_{12}$ linéaire ou ramifié, dans lequel se trouve(nt) éventuellement intercalé(s) un ou plusieurs hétéroatomes choisis parmi 0, N et S;
- les esters de vinyle de formule $R_5$-CO-O-CH = $CH_2$
  où $R_5$ représente un groupe alkyle en $C_4$ à $C_{12}$ linéaire ou ramifié ;
- les éthers d'alcool vinylique et d'alcool en $C_4$ à $C_{12}$,
- les N-alkyl en $C_4$ à $C_{12}$ acrylamides, tels que le N-octylacrylamide,
- et leurs mélanges.

Les monomères ayant une Tg inférieure ou égale à 20°C préférés sont l'acrylate d'isobutyle, l'acrylate d'éthyl-2 hexyle ou leurs mélanges en toutes proportions.

Chacune des première et deuxième séquences peut contenir, en proportion minoritaire, au moins un monomère constitutif de l'autre séquence.

Ainsi la première séquence peut contenir au moins un monomère constitutif de la deuxième séquence et inversement.

Chacune des première et/ou deuxième séquence, peu(ven)t comprendre, outre les monomères indiqués ci-dessus, un ou plusieurs autres monomères appelés monomères additionnels, différents des monomères principaux cités précédemment.

La nature et la quantité de ce ou ces monomères additionnels sont choisies de manière à ce que la séquence dans laquelle ils se trouvent ait la température de transition vitreuse désirée.

Ce monomère additionnel est par exemple choisi parmi :

- les monomères à insaturation(s) éthylénique(s) comprenant au moins une fonction amine tertiaire comme la 2-vinylpyridine, la 4-vinylpyridine, le méthacrylate de diméthylaminoéthyle, le méthacrylate de diéthylaminoéthyle, le diméthylaminopropyl méthacrylamide et les sels de ceux-ci,
- les méthacrylates de formule $CH_2 = C(CH_3)\text{-}COOR_6$
  dans laquelle $R_6$ représente un groupe alkyle linéaire ou ramifié, contenant de 1 à 4 atomes de carbone, tel qu'un groupe méthyle, éthyle, propyle ou isobutyle, ledit groupe alkyle étant substitué par un ou plusieurs substituants choisis parmi les groupes hydroxyle (comme le méthacrylate de 2-hydroxypropyle, le méthacrylate de 2-hydroxyéthyle) et les atomes d'halogènes (Cl, Br, I, F), tel que le méthacrylate de trifluoroéthyle,
- les méthacrylates de formule $CH_2 = C(CH_3)\text{-}COOR_9$,
  $R_9$ représentant un groupe alkyle en $C_6$ à $C_{12}$ linéaire ou ramifié, dans lequel se trouve(nt) éventuellement intercalé(s) un ou plusieurs hétéroatomes choisis parmi 0, N et S, ledit groupe alkyle étant substitué par un ou plusieurs substituants choisis parmi les groupes hydroxyle et les atomes d'halogènes (Cl, Br, I, F) ;
- les acrylates de formule $CH_2 = CHCOOR_{10}$,
  $R_{10}$ représentant un groupe alkyle en $C_1$ à $C_{12}$ linéaire ou ramifié substitué par un ou plusieurs substituants choisis parmi les groupes hydroxyle et les atomes d'halogène (Cl, Br, I et F), tel que l'acrylate de 2-hydroxypropyle et l'acrylate de 2-hydroxyéthyle, ou $R_{10}$ représente un alkyle en $C_1$ à $C_{12}$-O-POE (polyoxyéthylène) avec répétition du motif oxyéthylène de 5 à 10 fois, par exemple méthoxy-POE, ou $R_8$ représente un groupement polyoxyéthylèné comprenant de 5 à 10 motifs d'oxyde d'éthylène.

En particulier, la première séquence peut comprendre à titre de monomère additionnel :

- de l'acide (méth)acrylique, de préférence de l'acide acrylique,
- de l'acrylate de tertiobutyle
- les méthacrylates de formule $CH_2 = C(CH_3)\text{-}COOR_1$ dans laquelle $R_1$ représente un groupe alkyle non substitué, linéaire ou ramifié, contenant de 1 à 4 atomes de carbone, tel qu'un groupe méthyle, éthyle, propyle ou isobutyle,
- les (méth)acrylamides de formule :

  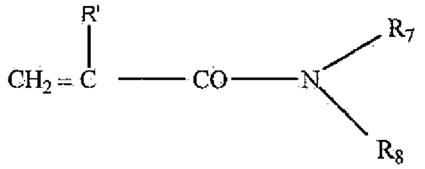

  où $R_7$ et $R_8$ identiques ou différents représentent chacun un atome d'hydrogène ou un groupe alkyle en $C_1$ à $C_{12}$ linéaire ou ramifié, tel qu'un groupe n-butyle, t-butyle, isopropyle, isohexyle, isooctyle, ou isononyle ; ou $R_7$ représente H et $R_8$ représente un groupement 1,1 -diméthyl-3-oxobutyl,
  et R' désigne H ou méthyle. Comme exemple de monomères, on peut citer le N-butylacrylamide, le N-t-butylacrylamide, le N-isopropylacrylamide, le N,N-diméthylacrylamide et le N,N-dibutylacrylamide,
- et leurs mélanges.

Le monomère additionnel peut représenter 0,5 à 30% en poids du poids du polymère. Selon un mode de mise en oeuvre, le polymère de l'invention ne contient pas de monomère additionnel.

De préférence, le polymère de l'invention comprend au moins des monomères acrylate d'isobornyle et méthacrylate d'isobornyle dans la première séquence et des monomères acrylate d'isobutyle et acide acrylique dans la deuxième séquence.

De préférence, le polymère comprend au moins des monomères acrylate d'isobornyle et méthacrylate d'isobornyle en proportion équivalente en poids dans la première séquence et des monomères acrylate d'isobutyle et acide acrylique dans la deuxième séquence.

De préférence, le polymère comprend au moins des monomères acrylate d'isobornyle et méthacrylate d'isobornyle en proportion équivalente en poids dans la première séquence, et des monomères acrylate d'isobutyle et acide acrylique dans la deuxième séquence, la première séquence représentant 70% en poids du polymère.

De préférence, le polymère comprend au moins des monomères acrylate d'isobornyle et méthacrylate d'isobornyle en proportion équivalente en poids dans la première séquence, et des monomères acrylate d'isobutyle et acide acrylique dans la deuxième séquence. De façon préférée, la séquence de Tg supérieure à 40°C représentant 70% en poids du polymère, et l'acide acrylique représentant 5% en poids du polymère.

Selon l'invention, la première séquence ne comprend pas de monomère additionnel.

Selon l'invention, la deuxième séquence comprend de l'acide acrylique à titre de monomère additionnel. En particulier, la deuxième séquence est avantageusement obtenue à partir d'un monomère acide acrylique et d'au moins un autre monomère ayant une Tg inférieure ou égale à 20°C.

Selon un mode de réalisation préféré, l'invention concerne une composition cosmétique pour le maquillage et/ou le soin des matières kératiniques comprenant, dans un milieu physiologiquement acceptable, au moins un copolymère éthylénique séquencé comprenant au moins un monomère acrylate de formule $CH_2 = CH\text{-}COOR_2$ dans laquelle $R_2$ représente un groupe cycloalkyle $C_8$ à $C_{12}$ et au moins un monomère méthacrylate de formule $CH_2 = C(CH_3)\text{-}COOR'_2$ dans laquelle $R'_2$ représente un groupe cycloalkyle $C_8$ à $C_{12}$, au moins un deuxième monomère acrylate de formule $CH_2 = CHCOOR_3$, dans laquelle $R_3$ représente un groupe alkyle non substitué en $C_1$ à $C_{12}$, linéaire ou ramifié, à l'exception du groupe tertiobutyle, et au moins un monomère acide acrylique,.

De façon préférée, le copolymère utilisé dans les compositions selon l'invention est obtenu à partir d'au moins un monomère méthacrylate d'isobomyle, d'au moins un monomère acrylate d'isobomyle, d'au moins un monomère acrylate d'isobutyle et d'au moins un monomère d'acide acrylique.

Avantageusement, le copolymère utilisé dans l'invention comprend de 50 à 80 % en poids de mélange méthacrylate/acrylate d'isobornyle, de 10 à 30 % en poids d'acrylate d'isobutyle et de 2 à 10 % en poids d'acide acrylique.

Le copolymère séquencé peut avantageusement comprendre plus de 2 % en poids de monomères acide acrylique, et notamment de 2 à 15 % en poids, par exemple de 3 à 15 % en poids, en particulier de 4 à 15 % en poids, voire de 4 à 10 % en poids de monomères acide acrylique, par rapport au poids total dudit copolymère.

Les monomères constitutifs de la deuxième séquence et leurs proportions sont choisis de telle sorte que la température de transition vitreuse de la deuxième séquence est inférieure ou égale à 20°C.

**Segment intermédiaire**

Le segment intermédiaire (également appelé séquence intermédiaire) relie la première séquence et la deuxième séquence du polymère utilisé selon la présente invention. Le segment intermédiaire résulte de la polymérisation :

i) du ou des premiers monomères, et éventuellement du ou des monomères additionnels, restant disponibles après leur polymérisation à un taux de conversion d'au maximum 90% pour former la première séquence,

ii) et du ou des deuxièmes monomères, et éventuellement du ou des monomères additionnels, ajoutés dans le mélange réactionnel.

La formation de la deuxième séquence est initiée lorsque les premiers monomères ne réagissent plus ou ne s'incorporent plus dans la chaîne polymérique soit parce qu'ils sont tous consommés soit parce que leur réactivité ne leur permet plus d'être.

Ainsi le segment intermédiaire comprend les premiers monomères disponibles, résultant d'un taux de conversion de ces premiers monomères inférieur ou égal à 90%, lors de l'introduction du ou des deuxièmes monomères lors de la synthèse du polymère.

Le segment intermédiaire du polymère séquencé est un polymère statistique (peut également être appelé une séquence statistique). C'est-à-dire qu'il comprend une répartition statistique du ou des premiers monomères et du ou des deuxièmes monomères ainsi que du ou des monomères additionnels éventuellement présents.

Ainsi, le segment intermédiaire est une séquence statistique, de même que la première séquence et la deuxième séquence si elles ne sont pas des homopolymères (c'est-à-dire si elles sont toutes deux formées à partir d'au moins deux monomères différents).

**Procédé de préparation du copolymère :**

Le copolymère éthylénique séquencé selon l'invention est préparé par polymérisation radicalaire libre, selon les techniques bien connues de ce type de polymérisation.

La polymérisation radicalaire libre est effectuée en présence d'un amorceur dont la nature est adaptée, de façon connue, en fonction de la température de polymérisation souhaitée et du solvant de polymérisation. En particulier, l'amorceur peut être choisi parmi les amorceurs à fonction peroxyde, les couples d'oxydoréduction, ou d'autres amorceurs de polymérisation radicalaire connus de l'homme de l'art.

En particulier, à titre d'amorceur à fonction peroxyde, on peut citer par exemple:

a. les péroxyesters, tel que le terbutyl-péroxyacétate, le perbenzoate de tertiobutyle, le tertbutyl péroxy-2-éthylhexanoate (Trigonox 21S d'Akzo Nobel), le 2,5-bis(2-éthylhexanoylpéroxy)-2,5-diméthylhexane (Trigonox 141 d'Akzo Nobel) ;
b. les péroxydicarbonates, tel que le di-isopropylpéroxydicarbonate ;
c. les péroxycetones, tel que le méthyléthylcétone peroxyde ;
d. hydropéroxydes, tel que l'eau oxygénée ($H_2O_2$), le terbutylhydropéroxyde ;
e. les péroxydes de diacyle, tel que l'acétyl péroxyde, le benzoyl peroxyde ;
f. les péroxydes de dialkyle, tel que le di-tertiobutyle péroxyde ;
g. les péroxydes inorganiques, tel que le péroxodisulfate de potassium ($K_2S_2O_8$):

A titre d'amorceur sous forme de couple d'oxydoréduction, on peut citer le couple thiosulfate de potassium + peroxodisulfate de potassium par exemple.

Selon un mode de réalisation préférée, l'amorceur est choisi parmi les peroxydes organiques comprenant de 8 à 30 atomes de carbone. De façon préférée, l'amorceur utilisé est le 2.5- Bis(2-éthylhexanoylperoxy)-2.5-diméthylhexane commercialisé sous la référence Trigonox® 141 par la société Akzo Nobel.

Le copolymère séquencé utilisé selon l'invention est préparé par polymérisation radicalaire libre et non par polymérisation contrôlée ou vivante. En particulier, la polymérisation du copolymère éthylénique séquencé est réalisée en l'absence d'agents de contrôle, et en particulier en l'absence d'agent de contrôle classiquement utilisés dans les procédés de polymérisation vivante ou contrôlée tels que les nitroxydes, les alcoxyamines, les dithioesters, les dithiocarbamates, les dithiocarbonates ou xanthates, les trithiocarbonates, les catalyseurs à base de cuivre, par exemple.

Comme indiqué précédemment, le segment intermédiaire est une séquence statistique, de même que la première séquence et la deuxième séquence si elles ne sont pas des homopolymères (c'est-à-dire si elles sont toutes deux formées à partir d'au moins deux monomères différents).

Le copolymère séquencé peut être préparé par polymérisation radicalaire libre, et en particulier par un procédé consistant à mélanger, dans un même réacteur, un solvant de polymérisation, un amorceur, au moins un monomère de transition vitreuse supérieure ou égale à 40°C, au moins un monomère de transition vitreuse inférieure ou égale à 20 °C selon la séquence suivante :

- on verse dans le réacteur, une partie du solvant de polymérisation et éventuellement une partie de l'amorceur et des monomères de la première coulée, mélange que l'on chauffe à une température de réaction comprise entre 60 et 120 °C,
- on verse ensuite, en une première coulée, ledit au moins un premier monomère de Tg supérieure ou égale à 40°C et éventuellement une partie de l'amorceur que l'on laisse à réagir pendant une durée T correspondant à un taux de conversion desdits monomères de 90 % maximum,
- on verse ensuite dans le réacteur, en une deuxième coulée, à nouveau de l'amorceur de polymérisation, ledit au moins un deuxième monomère de transition vitreuse inférieure ou égale à 20 °C, qu'on laisse réagir pendant une durée T' au bout de laquelle le taux de conversion desdits monomères atteint un plateau,
- on ramène le mélange réactionnel à température ambiante.

De façon préférée, le copolymère peut être préparé par polymérisation radicalaire libre, en particulier par un procédé consistant à mélanger, dans un même réacteur, un solvant de polymérisation, un amorceur, un monomère acide acrylique, au moins un monomère de transition vitreuse inférieure ou égale à 20 °C, au moins un monomère acrylate de formule $CH_2 = CH\text{-}COOR_2$ dans laquelle $R_2$ représente un groupe cycloalkyle $C_4$ à $C_{12}$, et au moins un monomère méthacrylate de formule $CH_2 = C(CH_3)\text{-}COOR'_2$ dans laquelle $R'_2$ représente un groupe cycloalkyle $C_4$ à $C_{12}$, selon la séquence d'étape suivante :

- on verse dans le réacteur, une partie du solvant de polymérisation et éventuellement une partie de l'amorceur et des monomères de la première coulée, mélange que l'on chauffe à une température de réaction comprise entre 60

et 120 °C,

- on verse ensuite, en une première coulée, ledit au moins monomère acrylate de formule $CH_2$ = CH-$COOR_2$ et ledit au moins monomère méthacrylate de formule $CH_2$ = $C(CH_3)$-COOR'$_2$ en tant que monomères de Tg supérieure ou égale à 40°C, et éventuellement une partie de l'amorceur que l'on laisse à réagir pendant une durée T correspondant à un taux de conversion desdits monomères de 90 % maximum,

- on verse ensuite dans le réacteur, en une deuxième coulée, à nouveau de l'amorceur de polymérisation, le monomère acide acrylique et ledit au moins monomère de transition vitreuse inférieure ou égale à 20 °C, qu'on laisse réagir pendant une durée T' au bout de laquelle le taux de conversion desdits monomères atteint un plateau,
- on ramène le mélange réactionnel à température ambiante.

Par solvant de polymérisation, on entend un solvant ou un mélange de solvants. En particulier, à titre de solvant de polymérisation utilisable on peut citer :

- les cétones liquides à température ambiante tels que méthyléthylcétone, méthylisobutylcétone, diisobutylcétone, l'isophorone, la cyclohexanone, l'acétane ;
- les éthers de propylène glycol liquides à température ambiante tels que le monométhyléther de propylène glycol, l'acétate de monométhyl éther de propylène glycol, le mono n-butyl éther de dipropylène glycol ;
- les esters à chaîne courte (ayant de 3 à 8 atomes de carbone au total) tels que l'acétate d'éthyle, l'acétate de méthyle, l'acétate de propyle, l'acétate de n-butyle, l'acétate d'isopentyle ;
- les éthers liquides à température ambiante tels que le diéthyléther, le diméthyléther ou le dichlorodiéthyléther ;
- les alcanes liquides à température ambiante tels que le décane, l'heptane, le dodécane, l'isododécane, le cyclohexane, l'isohexadécane ;
- les composés cycliques aromatiques liquides à température ambiante tels que le toluène et le xylène ; les aldéhydes liquides à température ambiante tels que le benzaldéhyde, l'acétaldéhyde et leurs mélanges.

Classiquement, le solvant de polymérisation est une huile volatile de point éclair inférieur à 80°C. Le point éclair est mesuré en particulier selon la Norme Iso 3679.

Le solvant de polymérisation peut être choisi notamment parmi l'acétate d'éthyle, l'acétate de butyle, les alcools tels que l'isopropanol, l'ethanol, les alcanes aliphatiques tels que l'isododécane et leurs mélanges. De préférence, le solvant de polymérisation est un mélange acétate de butyle et isopropanol ou l'isododécane.

Selon un autre mode de mise en oeuvre, le copolymère peut être préparé par polymérisation radicalaire libre selon un procédé de préparation, consistant à mélanger, dans un même réacteur, un solvant de polymérisation, un amorceur, au moins un monomère de transition vitreuse inférieure ou égale à 20 °C, et au moins un monomère de Tg supérieure ou égale à 40°C, selon la séquence d'étape suivante :

- on verse dans le réacteur, une partie du solvant de polymérisation et éventuellement une partie de l'amorceur et des monomères de la première coulée, mélange que l'on chauffe à une température de réaction comprise entre 60 et 120 °C,
- on verse ensuite, en une première coulée, ledit au moins un monomère de transition vitreuse inférieure ou égale à 20 °C et éventuellement une partie de l'amorceur que l'on laisse à réagir pendant une durée T correspondant à un taux de conversion desdits monomères de 90 % maximum,
- on verse ensuite dans le réacteur, en une deuxième coulée, à nouveau de l'amorceur de polymérisation, ledit au moins un monomère de Tg supérieure ou égale à 40°C, qu'on laisse réagir pendant une durée T' au bout de laquelle le taux de conversion desdits monomères atteint un plateau,
- on ramène le mélange réactionnel à température ambiante.

Selon un mode préféré de mise en oeuvre, le copolymère peut être préparé par polymérisation radicalaire libre selon un procédé de préparation, consistant à mélanger, dans un même réacteur, un solvant de polymérisation, un amorceur, un monomère acide acrylique, au moins un monomère de transition vitreuse inférieure ou égale à 20 °C, au moins un monomère de Tg supérieure ou égale à 40°C, et en particulier en tant que monomères de Tg supérieure ou égale à 40°C, au moins un monomère acrylate de formule $CH_2$ = CH-$COOR_2$ dans laquelle $R_2$ représente un groupe cycloalkyle $C_4$ à $C_{12}$, et au moins un monomère méthacrylate de formule $CH_2$ = $C(CH_3)$-COOR'$_2$ dans laquelle R'$_2$ représente un groupe cycloalkyle $C_4$ à $C_{12}$, selon la séquence d'étape suivante :

- on verse dans le réacteur, une partie du solvant de polymérisation et éventuellement une partie de l'amorceur et des monomères de la première coulée, mélange que l'on chauffe à une température de réaction comprise entre 60 et 120 °C,

- on verse ensuite, en une première coulée, le monomère acide acrylique et ledit au moins monomère de transition vitreuse inférieure ou égale à 20 °C et éventuellement une partie de l'amorceur que l'on laisse à réagir pendant une durée T correspondant à un taux de conversion desdits monomères de 90 % maximum,
- on verse ensuite dans le réacteur, en une deuxième coulée, à nouveau de l'amorceur de polymérisation, ledit au moins un monomère acrylate de formule $CH_2$ = CH-$COOR_2$ et ledit au moins un monomère méthacrylate de formule $CH_2$ = C($CH_3$)-COOR'$_2$, en tant que monomère de Tg supérieure ou égale à 40°C, qu'on laisse réagir pendant une durée T'au bout de laquelle le taux de conversion desdits monomères atteint un plateau,
- on ramène le mélange réactionnel à température ambiante.

La température de polymérisation est de préférence de l'ordre de 90 °C.
La durée de réaction après la deuxième coulée est de préférence comprise entre 3 et 6 heures.

**Distillation du solvant de synthèse**

Pour la mise en oeuvre du polymère séquencé dans une composition selon l'invention, et lorsque le polymère est préparé dans un solvant volatil ou une huile volatile ayant un point éclair inférieur à 80°C, il est nécessaire de procéder à une étape d'élimination totale ou partielle dudit solvant ou huile volatile. On procède en particulier par distillation, éventuellement sous vide, et ajout d'huile ester hydrocarbonée non volatile comprenant au moins 16 atomes de carbone et ayant une masse molaire inférieure à 650 g/moles.
Cette technique est connue de l'homme du métier et l'exemple 2 décrit ci-après illustre cette technique.
La distillation du solvant de synthèse (classiquement l'isododécane) est réalisée avec ajout simultané ou en présence dans le mélange avant la distillation d'une huile ester hydrocarbonée non volatile comprenant au moins 16 atomes de carbone et ayant une masse molaire inférieure à 650 g/mole. Cette étape est réalisée à chaud et éventuellement sous vide pour distiller un maximum d'isododécane (et plus généralement de solvant de synthèse), si celui-ci a été utilisé en tant que solvant de polymérisation, ou plus généralement pour distiller un maximum d'huile volatile dont le point éclair est inférieur à 80°C. L'huile ester non volatile peut également être ajoutée en partie ou intégralement au polymère dans le solvant volatil avant la distillation.
L'élimination de l'huile volatile de point éclair inférieur à 80°C (classiquement l'isododécane), permet de limiter la teneur de celle-ci dans la solution de copolymère séquencé et ainsi de réaliser une composition cosmétique contenant moins de 10% en poids d'isododécane (et plus généralement de solvant volatil) et de préférence moins de 5 % en poids d'isododécane (et plus généralement de solvant volatil), par rapport au poids total de la composition.
La composition selon l'invention comprend de préférence moins de 0,5 à 40 % en poids de copolymère éthylénique séquencé, et avantageusement de 1 à 40 % en poids, notamment de 2 à 30 % en poids, voire de 2 à 20 % en poids de matière active par rapport au poids total de la composition.

**Huile ester hydrocarbonée non volatile :**

La composition selon l'invention comprend une huile ester hydrocarbonée non volatile comprenant au moins 16 atomes de carbone et ayant une masse molaire inférieure à 650 g/mole.
Par « huile », on entend un composé non aqueux, liquide à température ambiante (25 °C) et pression atmosphérique (760 mm de Hg ; $1,013.10^5$ Pa).
Par "huile non volatile", on entend une huile restant sur les matières kératiniques à température ambiante et pression atmosphérique au moins plusieurs heures et ayant notamment une pression de vapeur inférieure à $10^{-3}$ mm de Hg (0,13 Pa). On peut également définir une huile non volatile comme ayant une vitesse d'évaporation telle que dans les conditions définies précédemment, la quantité évaporée au bout de 30 minutes est inférieure à 0,07mg/$cm^2$.
Par « huile hydrocarbonée », on entend une huile formée essentiellement, voire constituée, d'atomes de carbone et d'hydrogène, et éventuellement d'atomes d'oxygène, d'azote, et ne contenant pas d'atome de silicium ou de fluor. Par « huile ester hydrocarbonée », on entend une huile hydrocarbonée comprenant au moins un groupe ester.
La présence ou l'ajout de l'huile ester hydrocarbonée non volatile comprenant au moins 16 atomes de carbone et ayant une masse molaire inférieure à 650 g/mole permet notamment de substituer via la distillation, et donc de limiter la teneur dans la composition (voire de s'affranchir totalement) de la ou des huiles volatiles dont le point éclair est inférieur ou égal à 80°C, notamment celles ayant été utilisées en tant que solvant de polymérisation, en particulier telles que l'isododécane. En effet, ces huiles volatiles sont couramment utilisées en tant que procédé de polymérisation pour les copolymères présents dans la composition et comme cela a été indiqué précédemment, génèrent une sensation d'inconfort, de sécheresse et/ou de tiraillement sur les lèvres et imposent en outre des contraintes en termes de procédé de préparation de la composition, notamment lorsque le formulateur souhaite ajouter dans celle-ci des matières premières nécessitant de chauffer la composition, en particulier s'il est nécessaire de chauffer à une température supérieure au point éclair du solvant de polymérisation, et les huiles volatiles sont également à l'origine de contraintes en termes de

packaging, celui-ci devant alors être étanche.

Comme huile ester hydrocarbonée non volatile comprenant au moins 16 atomes de carbone et ayant une masse molaire inférieure à 650 g/mole, on peut notamment citer :

- les esters d'acide gras, en particulier de 4 à 22 atomes de carbone, et notamment d'acide octanoïque, d'acide heptanoïque, d'acide lanolique, d'acide oléique, d'acide laurique, d'acide stéarique comme le dioctanoate de propylène glycol, le monoisostéarate de propylène glycol, le diheptanoate de néopentylglycol,
- les esters de synthèse comme les huiles de formule $R_1COOR_2$ dans laquelle $R_1$ représente le reste d'un acide gras linéaire ou ramifié comportant de 4 à 40 atomes de carbone et $R_2$ représente une chaîne hydrocarbonée notamment ramifiée contenant de 4 à 40 atomes de carbone à condition que $R_1 + R_2$ soit ≥ 16, comme par exemple l'huile de Purcellin (octanoate de cétostéaryle), l'isononanoate d'isononyle, le benzoate d'alcool en $C_{12}$ à $C_{15}$, le palmitate d'éthyl 2-hexyle, le néopentanoate d'octyledodécyle, le stéarate d'octyl-2 dodécyle, l'érucate d'octyl-2 dodécyle, l'isostéarate d'isostéaryle, le benzoate d'octyl-2 dodécyle, des octanoates, décanoates ou ricinoléates d'alcools ou de polyalcools, le myristate d'isopropyle, le palmitate d'isopropyle, le stéarate de butyle, le laurate d'hexyle, le palmitate de 2-éthyl-hexyle, le laurate de 2-hexyl-décyle, le palmitate de 2-octyl-décyle, le myristate de 2-octyldodécyle, le succinate de 2-diéthyl-hexyle; de façon préférée, les esters de synthèse R1COOR2 préférés dans laquelle $R_1$ représente le reste d'un acide gras linéaire ou ramifié comportant de 4 à 40 atomes de carbone et $R_2$ représente une chaîne hydrocarbonée notamment ramifiée contenant de 4 à 40 atomes de carbone sont tels que R1 et R2 soit ≥ 20 ;
- les esters hydroxylés comme le lactate d'isostéaryle, l'octylhydroxystéarate, l'hydroxystéarate d'octyldodécyle, le diisostéarylmalate, le stéarate de glycérine; le diisononanoate de diéthylèneglycol ; et
- les esters du pentaérythritol ; les esters d'acides aromatiques et d'alcools comprenant 4 à 22 atomes de carbone.

Selon un mode de réalisation, huile ester hydrocarbonée non volatile comprenant au moins 16 atomes de carbone et ayant une masse molaire inférieure à 650 g/mole ne porte pas de groupe hydroxyle (OH) libre.

Selon un mode de réalisation préféré, l'huile ester hydrocarbonée non volatile comprend au moins 20 atomes de carbone a une masse molaire inférieure à 650 g/mole.

Selon un mode de réalisation préféré, l'huile ester hydrocarbonée non volatile comprenant au moins 16 atomes de carbone a une masse molaire inférieure à 600g/mole.

L'huile ester hydrocarbonée non volatile peut notamment comprendre une huile hydrocarbonée, linéaire ou ramifiée ayant une masse molaire comprise entre 100 et 650 g/mole et plus particulièrement entre 200 et 600 g/mole.

Le choix de ces huiles non volatiles permet en effet d'optimiser la quantité d'huile volatile dont le point éclair est inférieur ou égal à 80°C évaporée, c'est-à-dire de distiller la majeure partie du solvant de polymérisation (tel que l'isododécane), voire essentiellement tout le solvant de polymérisation. (c'est-à-dire d'huile volatile dont le point éclair est inférieure ou égal à 80°C), celui-ci ne subsistant qu'à l'état de trace dans la composition.

L'utilisation d'autres solvants ne permet pas d'éliminer suffisamment d'huile volatile dont le point éclair est inférieur ou égal à 80°C. Au cours de la distillation, le mélange devient très visqueux et non manipulable et il n'est plus possible de continuer la distillation.

Selon un mode de réalisation préféré, l'huile ester hydrocarbonée non volatile comprenant au moins 16 atomes de carbone (de préférence au moins 20 atomes de carbone) et ayant une masse molaire inférieure à 650 g/mole est le néopentanoate d'octyledodécyle (notamment le néopentanoate de 2-octyledodécyle). Cette huile ester permet en effet de distiller la totalité de l'huile volatile dont le point éclair est inférieur ou égal à 80°C.

La composition selon l'invention peut comprendre de 2 à 80% en poids d'huile non volatile, notamment de 5 à 70 %, par rapport au poids total de la composition. Selon un mode de réalisation préféré, le ratio en poids de copolymère éthylénique séquencé par rapport au poids d'huile ester hydrocarbonée non volatile est inférieur à 1, de préférence inférieur à 0,75 , ou mieux inférieur à 0,5.

## Huile non volatile additionnelle

La composition selon l'invention peut avantageusement comprendre au moins une autre huile non volatile additionnelle, différente de ladite huile ester hydrocarbonée comprenant au moins 16 atomes de carbone et ayant une masse molaire inférieure à 650 g/mole,

Cette huile additionnelle peut être choisie parmi toutes les huiles cosmétiquement acceptables, notamment les huiles minérales, végétales, synthétiques ; en particulier les huiles hydrocarbonées et/ou siliconées et/ou fluorées, volatiles ou non volatiles et leurs mélanges.

Au sens de la présente invention, on entend par « huile siliconée », une huile comprenant au moins un atome de silicium, et notamment au moins un groupe Si-O.

On entend par "huile fluorée", une huile comprenant au moins un atome de fluor.

Plus précisément, par « huile hydrocarbonée », on entend une huile formée principalement, voir constituée, d'atomes de carbone et d'hydrogène, et ne contenant pas d'atome de silicium ou de fluor, et comprenant éventuellement une ou plusieurs fonctions choisies parmi les fonctions hydroxyle, ester, éther ou carboxylique.

A titre d'exemple d'huile hydrocarbonée non volatile additionnelle on peut citer :

1/ les hydrocarbures linéaires ou ramifiés, d'origine minérale ou synthétique tels que :

- l'huile de paraffine ou ses dérivés,
- la vaseline,
- les polybutylènes tels que L'INDOPOL H-100 (de masse molaire ou MW=965 g/mol), L'INDOPOL H-300 (MW=1340 g/mol), L'INDOPOL H-1500 (MW=2160g/mol) commercialisés ou fabriqués par la société AMOCO,
- les polyisobutylènes hydrogénés tels que le Parléam® commercialisé par la société NIPPON OIL FATS, le PANALANE H-300 E commercialisé ou fabriqué par la société AMOCO (MW=1340 g/mol), le VISEAL 20000 commercialisé ou fabriqué par la société SYNTEAL (MW=6000 g/mol), le REWOPAL PIB 1000 commercialisé ou fabriqué par la société WITCO (MW=1000 g/mol),
- les polydécènes et les polydécènes hydrogénés tels que : le PURESYN 10 (MW=723 g/mol), le PURESYN 150 (MW=9200 g/mol) commercialisés ou fabriqués par la société MOBIL CHEMICALS,

2/ les copolymères de la vinylpyrrolidone tels que : le copolymère vinylpyrrolidone/1-héxadécène, ANTARON V-216 commercialisé ou fabriqué par la société ISP (MW=7300 g/mol),

3/ les huile ester hydrocarbonée ayant une masse molaire supérieure à 650 g/mole, tels que

- les esters d'acides gras linéaires ayant un nombre total de carbone allant de 35 à 70 comme le tétrapélargonate de pentaérythrityle (MW=697 g/mol),
- les esters hydroxylés tels que le triisostéarate de polyglycérol-2 (MW=965 g/mol),
- les esters aromatiques tels que le tridécyl trimellitate (MW=757 g/mol),
- les esters d'alcool gras ou d'acides gras ramifiés en $C_{24}$-$C_{28}$ tels que ceux décrits dans la demande EP-A-0 955 039, et notamment le citrate de triisoarachidyle (MW=1033,76 g/mol), le tétraisononanoate de pentaérythrityle (MW=697 g/mol), le triisostéarate de glycéryle (MM=891 g/mol), le tri décyl-2 tétradécanoate de glycéryle (MW=1143 g/mol), le tétraisostéarate de pentaérythrityle (MW=1202 g/mol), le tétraisostéarate de polyglycéryle -2 (MW=1232 g/mol) ou encore le tétra décyl-2 tétradécanoate de pentaérythrityle (MW=1538 g/mol),
- un polyester résultant de l'estérification d'au moins un triglycéride d'acide(s) carboxylique(s) hydroxylé(s) par un acide monocarboxylique aliphatique et par un acide dicarboxylique aliphatique, éventuellement insaturé comme l'huile de ricin d'acide succinique et d'acide isostéarique commercialisée sous la référence Zénigloss par Zénitech,
- les esters résultant de l'estérification d'un polyol et d'un dimer diacide tel que le copolymère d'isostéarate de polyglyceryle-2 dimerdilinoléate (Hailucent ISDA),
- les esters de dimère diol et de dimère diacide de formule générale HO-$R^1$-(-OCO-$R^2$-COO-$R^1$-)$_h$-OH, dans laquelle :

  $R^1$ représente un reste de dimère diol obtenu par hydrogénation du diacide dilinoléique
  $R^2$ représente un reste de diacide dilinoléique hydrogéné, et
  h représente un entier variant de 1 à 9,
  notamment les esters de diacides dilinoléiques et de dimères diols dilinoléiques commercialisés par la société NIPPON FINE CHEMICAL sous la dénomination commerciale LUSPLAN DD-DA5® et DD-DA7®,

4/ les huiles siliconées telles que les silicones phénylées (également appelée huile siliconée phénylée) comme la BELSIL PDM 1000 de la société WACKER (MW=9000 g/mol), les phényl triméthicones (telles que la phényl triméthicone vendue sous le nom commercial DC556 par Dow Corning), les phényl diméthicones, les phényl triméthylsiloxy diphényl siloxanes, les diphényl diméthicones, les diphényl méthyldiphényl trisiloxanes, les polydiméthylsiloxanes (PDMS) non volatiles, les polydiméthylsiloxanes comportant des groupements alkyle ou alcoxy, pendant et/ou en bout de chaîne siliconée, groupements ayant chacun de 2 à 24 atomes de carbone ;

5/ les huiles d'origine végétale telles que l'huile de sésame (MW=820 g/mol),

- et leurs mélanges.

De façon préférée, la composition selon l'invention contient avantageusement de 1 à 80%, en poids, en particulier de 5 à 70 % en poids, de préférence de 10 à 65 % en poids total d'huile additionnelle non volatile, par rapport au poids total de la composition.

**Huiles dont le point éclair est inférieur ou égal à 80°C :**

La composition contient moins de 10% en poids d'huile volatile dont le point éclair est inférieur ou égal à 80°C, ou mieux moins de 5 % par rapport au poids total de la composition ou encore est exempte d'huile volatile dont le point éclair est inférieur ou égal à 80°C, telles que l'isododécane.

Le point éclair est en particulier mesuré selon la Norme iso 3679.

Par " huile volatile", on entend au sens de l'invention une huile susceptible de s'évaporer au contact des matières kératiniques en moins d'une heure, à température ambiante et pression atmosphérique (760mm de Hg; 1,013.$10^5$ Pa). Le ou les solvants organiques volatils et les huiles volatiles de l'invention sont des solvants organiques et des huiles cosmétiques volatiles, liquides à température ambiante, ayant une pression de vapeur non nulle, à température ambiante et pression atmosphérique, allant en particulier de 0,13 Pa à 40000 Pa ($10^{-3}$ à 300 mm de Hg), en particulier allant de 1,3 Pa à 13000 Pa (0,01 à 100 mm de Hg), et plus particulièrement allant de 1,3 Pa à 1300 Pa (0,01 à 10 mm de Hg).

Ces huiles peuvent être des huiles hydrocarbonées, des huiles siliconées, des huiles fluorées, ou leurs mélanges.

En particulier, comme huile volatile dont le point éclair est inférieur ou égal à 80°C, on peut citer :

Des huiles hydrocarbonées volatiles telles les huiles hydrocarbonées ayant de 8 à 14 atomes de carbone, et notamment les alcanes ramifiés en $C_8$-$C_{14}$ comme les isoalcanes en $C_8$-$C_{14}$. d'origine pétrolière (appelées aussi isoparaffines) comme l'isododécane (encore appelé 2,2,4,4,6-pentaméthylheptane), l'isodécane, et par exemple les huiles vendues sous les noms commerciaux d'Isopars' ou de Permetyls, et leurs mélanges. De préférence, le solvant volatil est choisi parmi les huiles volatiles hydrocarbonées ayant de 8 à 14 atomes de carbone et leurs mélanges.

Comme autres huiles hydrocarbonés volatiles dont le point éclair est inférieur ou égal à 80°C, on peut également citer, les cétones liquides à température ambiante tels que méthyléthylcétone, l'acétone; les esters à chaîne courte (ayant de 3 à 8 atomes de carbone au total) tels que l'acétate d'éthyle, l'acétate de méthyle, l'acétate de propyle, l'acétate de n-butyle ; les éthers liquides à température ambiante tels que le diéthyléther, le diméthyléther ou le dichlorodiéthyléther; les alcools et notamment les monoalcools inférieurs linéaires ou ramifiés ayant de 2 à 5 atomes de carbone comme l'éthanol, l'isopropanol ou le n-propanol.

Selon un mode de réalisation, la composition selon l'invention contient au moins une huile volatile dont le point éclair est supérieur à 80°C, telle que l'isohexadécane.

**2. Corps gras solides**

De façon avantageuse, la composition selon l'invention comprend au moins un corps gras solide choisi parmi les corps gras pâteux et/ou les cires.

**Corps gras pâteux**

Par "corps gras pâteux" (également appelé corps gras pâteux) au sens de la présente invention, on entend un composé gras lipophile à changement d'état solide/liquide réversible, présentant à l'état solide une organisation cristalline anisotrope, et comportant à la température de 23°C une fraction liquide et une fraction solide.

En d'autres termes, la température de fusion commençante du composé pâteux peut être inférieure à 23°C. La fraction liquide du composé pâteux mesurée à 23°C peut représenter 9 à 97% en poids du composé. Cette fraction liquide à 23 °C représente de préférence entre 15 et 85%, de préférence encore entre 40 et 85% en poids.

Au sens de l'invention, la température de fusion correspond à la température du pic le plus endothermique observé en analyse thermique (DSC) telle que décrite dans la norme ISO 11357-3 ; 1999. Le point de fusion d'un pâteux ou d'une cire peut être mesuré à l'aide d'un calorimètre à balayage différentiel (DSC), par exemple le calorimètre vendu sous la dénomination « MDSC 2920 » par la société TA Instruments.

Le protocole de mesure est le suivant :

Un échantillon de 5 mg de pâteux ou de cire (selon le cas) disposé dans un creuset est soumis à une première montée en température allant de -20 °C à 100 °C, à la vitesse de chauffe de 10 °C/minute, puis est refroidi de 100 °C à -20 °C à une vitesse de refroidissement de 10 °C/minute et enfin soumis à une deuxième montée en température

allant de -20 °C à 100 °C à une vitesse de chauffe de 5 °C/minute. Pendant la deuxième montée en température, on mesure la variation de la différence de puissance absorbée par le creuset vide et par le creuset contenant l'échantillon de pâteux ou de cire en fonction de la température. Le point de fusion du composé est la valeur de la température correspondant au sommet du pic de la courbe représentant la variation de la différence de puissance absorbée en fonction de la température.

La fraction liquide en poids du composé pâteux à 23°C est égale au rapport de l'enthalpie de fusion consommée à 23°C sur l'enthalpie de fusion du composé pâteux.

L'enthalpie de fusion du composé pâteux est l'enthalpie consommée par le composé pour passer de l'état solide à l'état liquide. Le composé pâteux est dit à l'état solide lorsque l'intégralité de sa masse est sous forme solide cristalline. Le composé pâteux est dit à l'état liquide lorsque l'intégralité de sa masse est sous forme liquide.

L'enthalpie de fusion du composé pâteux est égale à l'aire sous la courbe du thermogramme obtenu à l'aide d'un calorimètre à balayage différentiel (D. S. C), tel que le calorimètre vendu sous la dénomination MDSC 2920 par la société TA instrument, avec une montée en température de 5 ou 10°C par minute, selon la norme ISO 11357-3:1999. L'enthalpie de fusion du composé pâteux est la quantité d'énergie nécessaire pour faire passer le composé de l'état solide à l'état liquide. Elle est exprimée en J/g.

L'enthalpie de fusion consommée à 23°C est la quantité d'énergie absorbée par l'échantillon pour passer de l'état solide à l'état qu'il présente à 23°C constitué d'une fraction liquide et d'une fraction solide.

La fraction liquide du composé pâteux mesurée à 32°C représente de préférence de 30 à 100% en poids du composé, de préférence de 50 à 100%, de préférence encore de 60 à 100% en poids du composé. Lorsque la fraction liquide du composé pâteux mesurée à 32°C est égale à 100%, la température de la fin de la plage de fusion du composé pâteux est inférieure ou égale à 32°C.

La fraction liquide du composé pâteux mesurée à 32°C est égale au rapport de l'enthalpie de fusion consommée à 32°C sur l'enthalpie de fusion du composé pâteux. L'enthalpie de fusion consommée à 32°C est calculée de la même façon que l'enthalpie de fusion consommée à 23°C.

Le composé pâteux est de préférence choisi parmi les composés synthétiques et les composés d'origine végétale. Un composé pâteux peut être obtenu par synthèse à partir de produits de départ d'origine végétale.

Le composé pâteux est avantageusement choisi parmi

- la lanoline et ses dérivés
- les éthers de polyol choisi parmi les éthers de pentaérythritol et de polyalkylène glycol, les éthers d'alcool gras et de sucre, et leurs mélanges l'éther pentaérythritol et de polyéthylène glycol comportant 5 motifs oxyéthylénés (5 OE) (nom CTFA : PEG-5 Pentaerythrityl Ether), l'éther de pentaérythritol et de polypropylène glycol comportant 5 motifs oxypropylénés (5 OP) (nom CTFA : PPG-5 Pentaerythrityl Ether), et leurs mélanges et plus spécialement le mélange PEG-5 Pentaerythrityl Ether, PPG-5 Pentaerythrityl Ether et huile de soja, commercialisé sous la dénomination « Lanolide » par la société Vevy, mélange où les constituants se trouvent dans un rapport en poids 46/46/8 : 46 % de PEG-5 Pentaerythrityl Ether, 46 % de PPG-5 Pentaerythrityl Ether et 8 % d'huile de soja.
- les composés siliconés polymères ou non
- les composés fluorés polymères ou non
- les polymères vinyliques, notamment:
  - les homopolymères et les copolymères d'oléfines
  - les homopolymères et copolymères de diènes hydrogénés
  - les oligomères linéaires ou ramifiés, homo ou copolymères de (méth)acrylates d'alkyles ayant de préférence un groupement alkyle en $C_8$-$C_{30}$
  - les oligomères homo et copolymères d'esters vinyliques ayant des groupements alkyles en $C_8$-$C_{30}$
  - les oligomères homo et copolymères de vinyléthers ayant des groupements alkyles en $C_8$-$C_{30}$,
- les polyéthers liposolubles résultant de la polyéthérification entre un ou plusieurs diols en C2-C100, de préférence en C2-C50,
- les esters,
- et/ou leurs mélanges.

Le composé pâteux est de préférence un polymère, notamment hydrocarboné.

Parmi les polyéthers liposolubles, on préfère en particulier les copolymères d'éthylène-oxyde et/ou de propylène-oxyde avec des alkylènes-oxydes à longue chaîne en C6-C30, de préférence encore tels que le rapport pondéral de l'éthylène-oxyde et/ou de propylène-oxyde avec alkylènes-oxydes dans le copolymère est de 5:95 à 70:30. Dans cette famille, on citera notamment les copolymères tels que les alkylènes-oxydes à longue chaîne sont disposés en

blocs ayant un poids moléculaire moyen de 1.000 à 10.000, par exemple un copolymère bloc de polyoxyethylène/polydodécyle glycol tel que les éthers de dodécanediol (22 mol) et de polyéthylène glycol (45 OE) commercialisés sous la marque ELFACOS ST9 par Akzo Nobel.

Parmi les esters, on préfère notamment :

- les esters d'un glycérol oligomère, notamment les esters de diglycérol, en particulier les condensats d'acide adipique et de glycérol, pour lesquels une partie des groupes hydroxyles des glycérols ont réagi avec un mélange d'acides gras tels que l'acide stéarique, l'acide caprique, l'acide stéarique et l'acide isostéarique et l'acide 12-hydroxystéarique, à l'image notamment de ceux commercialisé sous la marque Softisan 649 par la société Sasol
- le propionate d'arachidyle commercialisé sous la marque Waxenol 801 par Alzo,
- les esters de phytostérol,
- les triglycérides d'acides gras et leurs dérivés
- les esters de pentaérythritol
- les polyesters non réticulés résultant de la polycondensation entre un acide dicarboxylique ou un polyacide carboxylique linéaire ou ramifié en C4-C50 et un diol ou un polyol en C2-C50,

les esters aliphatiques d'ester résultant de l'estérification d'un ester d'acide hydroxycarboxylique aliphatique par un acide carboxylique aliphatique. De préférence, l'acide carboxylique aliphatique comprend de 4 à 30 et de préférence de 8 à 30 atomes de carbone. Il est de préférence choisi parmi l'acide héxanoïque, l'acide heptanoïque, l'acide octanoïque, l'acide éthyl-2 héxanoïque, l'acide nonanoïque, l'acide décanoïque, l'acide undécanoïque, acide dodécanoïque, l'acide tridécanoïque, l'acide tétradécanoïque, l'acide pentadécanoïque, l'acide héxadécanoïque, l'acide héxyldécanoïque, l'acide heptadécanoïque, l'acide octadécanoïque, l'acide isostéarique, l'acide nonadécanoïque, l'acide eicosanoïque, l'acide isoarachidique, l'acide octyldodécanoïque, l'acide benéicosanoïque, l'acide docosanoïque, et leurs mélanges. L'acide carboxylique aliphatique est de préférence ramifié. L'ester d'acide hydroxy carboxylique aliphatique est avantageusement issu d'un acide carboxylique aliphatique hydroxylé comportant de 2 à 40 atomes de carbone, de préférence de 10 à 34 atomes de carbone et mieux de 12 à 28 atomes de carbone, et de 1 à 20 groupes hydroxyle, de préférence de 1 à 10 groupes hydroxyle et mieux de 1 à 6 groupes hydroxyle. L'ester d'acide hydroxy carboxylique aliphatique est choisi parmi :

a) les esters partiels ou totaux d'acides monocarboxyliques aliphatiques mono hydroxylés linéaires, saturés ;
b) les esters partiels ou totaux d'acides monocarboxyliques aliphatiques mono hydroxylés insaturés ;
c) les esters partiels ou totaux de polyacides carboxyliques aliphatiques mono hydroxylés saturés ;
d) les esters partiels ou totaux de polyacides carboxyliques aliphatiques poly hydroxylés saturés ;
e) les esters partiels ou totaux de polyols aliphatiques en $C_2$ à $C_{16}$ ayant réagi avec un mono ou un poly acide carboxylique aliphatique mono ou poly hydroxylé,

et leurs mélanges.

- les esters de dimère diol et dimère diacide, le cas échéant, estérifiés sur leur(s) fonction(s) alcool(s) ou acide(s) libre(s) par des radicaux acides ou alcools, notamment les esters dimer dilinoleate, de tels esters peuvent être notamment choisis parmi les esters de nomenclature INCI suivante : le bis-béhényl/isostéaryl/phytostéryl dimerdilinoléyle dimerdilinoléate (Plandool G), le phytostéryl isostéaryl dimerdilinoléate (Lusplan PI-DA, Lusplan PHY/IS-DA), le phytostéryl/isostéryl/cétyl/stéaryl/béhényl dimerdilinoléate (Plandool H ou Plandool S), et leurs mélanges.
- les esters de rosinate hydrogénée, tel que les dimères dilinoleyl de rosinate hydrogéné (Lusplan DD-DHR ou DD-DHR de Nippon Fine Chemical)
- et leurs mélanges.

De façon avantageuse, le (ou les) composé pâteux représente de préférence 0,1 à 80%, mieux 0,5 à 60%, mieux 1 à 30% et mieux encore 1 à 20% en poids par rapport au poids total de la composition.

## Cire(s)

Selon un mode de réalisation préféré, la composition selon l'invention comprend au moins une cire.

La cire considérée dans le cadre de la présente invention est d'une manière générale un composé lipophile, solide à température ambiante (25 °C), à changement d'état solide/liquide réversible, ayant un point de fusion supérieur ou égal à 30 °C pouvant aller jusqu'à 200 °C et notamment jusqu'à 120 °C.

En particulier, les cires convenant à l'invention peuvent présenter un point de fusion supérieur ou égal à 45 °C, et en particulier supérieur ou égal à 55 °C.

Les cires susceptibles d'être utilisées dans les compositions selon l'invention sont choisies parmi les cires, solides, à température ambiante d'origine animale, végétale, minérale ou de synthèse et leurs mélanges.

A titre illustratif des cires convenant à l'invention, on peut notamment citer les cires hydrocarbonées comme la cire d'abeille, la cire de lanoline, et les cires d'insectes de Chine, la cire de son de riz, la cire de Carnauba, la cire de Candellila, la cire d'Ouricury, la cire d'Alfa, la cire de berry, la cire de shellac, la cire du Japon et la cire de sumac; la cire de montan, les cires d'orange et de citron, les cires microcristallines, les paraffines et l'ozokérite; les cires de polyéthylène, les cires obtenues par la synthèse de Fisher-Tropsch et les copolymères cireux ainsi que leurs esters.

On peut aussi citer des cires obtenues par hydrogénation catalytique d'huiles animales ou végétales ayant des chaînes grasses, linéaires ou ramifiées, en $C_8$-$C_{32}$. Parmi celles-ci, on peut notamment citer l'huile de jojoba isomérisée telle que l'huile de jojoba partiellement hydrogénée isomérisée trans fabriquée ou commercialisée par la société DESERT WHALE sous la référence commerciale Iso-Jojoba-50®, l'huile de tournesol hydrogénée, l'huile de ricin hydrogénée, l'huile de coprah hydrogénée, l'huile de lanoline hydrogénée, et le tétrastéarate de di-(triméthylol-1,1,1 propane) vendu sous la dénomination de Hest 2T-4S® par la société HETERENE.

On peut encore citer les cires de silicone ($C_{30-45}$ ALKYL DIMETHICONE), les cires fluorées.

On peut également utiliser les cires obtenues par hydrogénation d'huile de ricin estérifiée avec l'alcool cétylique vendues sous les dénominations de Phytowax ricin 16L64® et 22L73® par la société SOPHIM. De telles cires sont décrites dans la demande FR-A- 2792190.

Comme cire, on peut utiliser un (hydxoxystéaryloxy)stéarate d'alkyle en $C_{20}$-$C_{40}$ (le groupe alkyle comprenant de 20 à 40 atomes de carbone), seul ou en mélange.

Une telle cire est notamment vendue sous les dénominations « Kester Wax K 82 P® », « Hydroxypolyester K 82 P® » et « Kester Wax K 80 P® » par la société KOSTER KEUNEN.

Comme micro cires pouvant être utilisées dans les compositions selon l'invention, on peut citer notamment les micro cires de carnauba telles que celle commercialisée sous la dénomination de MicroCare 350® par la société MICRO POWDERS, les micro cires de cire synthétique telles que celle commercialisée sous la dénomination de MicroEase 114S® par la société MICRO POWDERS, les micro cires constituées d'un mélange de cire de carnauba et de cire de polyéthylène telles que celles commercialisées sous les dénominations de Micro Care 300® et 310® par la société MICRO POWDERS, les micro cires constituées d'un mélange de cire de carnauba et de cire synthétique telles que celle commercialisée sous la dénomination Micro Care 325® par la société MICRO POWDERS, les micro cires de polyéthylène telles que celles commercialisées sous les dénominations de Micropoly 200®, 220®, 220L® et 250S® par la société MICRO POWDERS et les micro cires de polytétrafluoroéthylène telles que celles commercialisées sous les dénominations de Microslip 519® et 519 L® par la société MICRO POWDERS.

La composition selon l'invention peut comprendre une teneur en cires allant de 0,1 à 30 % en poids par rapport au poids total de la composition, en particulier elle peut en contenir de 0,5 à 20 %, plus particulièrement de 1 à 15 %.

## Milieu Physiologiquement acceptable

Par « milieu physiologiquement acceptable », on entend un milieu compatible avec les matières kératiniques, comme les huiles ou les solvants organiques couramment employés dans les compositions cosmétiques.

Le milieu physiologiquement acceptable de la composition selon l'invention peut également comprendre un ou plusieurs solvants organiques, physiologiquement acceptables (tolérance, toxicologie et toucher acceptables).

## Résine tackifiante

Selon un mode de réalisation particulièrement avantageux, La composition selon l'invention comprend au moins une résine choisie parmi la colophane, les dérivés de colophane, les résines hydrocarbonées et leurs mélanges.

Ce mode de réalisation particulier permet notamment l'obtention d'une composition de maquillage et/ou de soin matières kératiniques, et notamment des lèvres et/ou de la peau, permettant d'obtenir un dépôt (notamment de maquillage) épais et homogène se déposant facilement sur les matières kératiniques (notamment sur la peau et/ou les lèvres), et présentant une tenue de la couleur et de la brillance satisfaisantes, à l'application et dans le temps, notamment 1 heure après l'application.

De plus, le dépôt obtenu est confortable (pas de sensation de tiraillement ou de sécheresse).

Selon ce mode de réalisation, la composition est de préférence sous forme solide et comprend au moins un corps gras solide. En particulier, elle peut se présenter sous la forme d'un rouge à lèvres en stick.

La résine utilisée dans la composition selon l'invention (également appelée résine tackifiante) a de préférence un poids moléculaire moyen en nombre inférieure ou égale à 10 000 g/mol, notamment allant de 250 à 10 000 g/mol de préférence inférieure ou égale à 5 000 g/mol, notamment allant de 250 à 5 000 g/mol, mieux, inférieure ou égale à 2 000 g/mol notamment allant de 250 à 2 000 g/mol et encore mieux inférieure ou égale à 1 000 g/mol notamment allant de 250 à 1 000 g/mol.

On détermine les poids moléculaires moyens en nombre (Mn) par chromatographie liquide par perméation de gel (solvant THF, courbe d'étalonnage établie avec des étalons de polystyrène linéaire, détecteur réfractométrique).

La résine de la composition selon l'invention est avantageusement une résine dite tackifiante. De telles résines sont notamment décrites dans le Handbook of Pressure Sensitive Adhesive, edited by Donatas Satas, 3rd ed., 1989, p. 609-619.

La résine de la composition selon l'invention est choisie parmi la colophane, les dérivés de colophane, les résines hydrocarbonées et leurs mélanges.

De préférence, la résine est une résine hydrocarbonée indénique, pouvant éventuellement être hydrogénée, et de préférence étant hydrogénée.

La colophane est un mélange comprenant majoritairement des acides organiques appelés acides de colophane (principalement des acides de type abiétique et de type pimarique).

Il existe trois types de colophane : la colophane ("gum rosin") obtenue par incision sur les arbres vivants, la colophane de bois ("wood rosin") qui est extraite des souches ou du bois de pins, et l'huile de tall ("tall oil rosin") qui est obtenue d'un sous-produit provenant de la production du papier.

Les dérivés de colophane peuvent être issus en particulier de la polymérisation, de l'hydrogénation et/ou de l'estérification (par exemple avec des alcools polyhydriques tels que l'éthylène glycol, le glycérol, le pentaérhytritol) des acides de colophanes. On peut citer par exemple les esters de colophanes commercialisés sous la référence FORAL 85, PENTALYN H et STAYBELITE ESTER 10 par la société HERCULES ; SYLVATAC 95 et ZONESTER 85 par la société ARIZONA CHEMICAL ou encore UNIREZ 3013 par la société UNION CAMP.

Les résines hydrocarbonées sont choisies parmi les polymères de faible poids moléculaire qui peuvent être classifiées, selon le type de monomère qu'elles comprennent, en :

- résines hydrocarbonées indéniques, notamment telles que les résines issues de la polymérisation en proportion majoritaire de monomère indène et en proportion minoritaire de monomère choisi parmi le styrène, le méthylindène, le méthylstyrène et leurs mélanges. Ces résines pouvant éventuellement être hydrogénées. Ces résines peuvent présenter un poids moléculaire allant de 290 à 1150 g/mol. Comme exemples de résines indéniques, on peut citer celles commercialisées sous la référence ESCOREZ 7105 par la société Exxon Chem., NEVCHEM 100 et NEVEX 100 par la société Neville Chem., NORSOLENE S105 par la société Sartomer, PICCO 6100 par la société Hercules et RESINALL par la société Resinall Corp., ou les copolymères indène/méthylstyrène/styrène hydrogéné commercialisées sous la dénomination « REGALITE » par la société Eastman Chemical, en particulier REGALITE R 1100, REGALITE R 1090, REGALITE R-7100, REGALITE R1010 HYDROCARBON RESIN, REGALITE R1125 HYDROCARBON RESIN.
- les résines aliphatiques de pentanediène telles que celle issues de la polymérisation majoritairement du monomères 1,3-pentanediène (trans ou cis pipérylène) et de monomère minoritaire choisi parmi l'isoprène, le butène, le 2-méthyl-2-butène, le pentène, le 1,4-pentanediène et leurs mélanges. Ces résines peuvent présenter un poids moléculaire allant de 1000 à 2500 g/mol. De telles résines du 1,3-pentanediène sont commercialisées par exemple sous les références PICCOTAC 95 par la société Eastman Chemical, ESCOREZ 1304 par la société Exxon Chemicals, NEVTAC 100 par la société Neville Chem. ou WINGTACK 95 par la société Goodyear;
- les résines mixtes de pentanediène et d'indène, qui sont issues de la polymérisation d'un mélange de monomères de pentanediène et d'indéne tels que ceux décrits ci-dessus, comme par exemple les résines commercialisées sous la référence ESCOREZ 2101 par la société Exxon Chemicals., NEVPENE 9500 par la société Neville Chem., HERCOTAC 1148 par la société Hercules., NORSOLENE A 100 par la société Sartomer, WINGTACK 86, WINGTACK EXTRA et WINGTACK PLUS par la société Goodyear,
- les résines diènes des dimères du cyclopentanediène telles que celles issues de la polymérisation de premier monomère choisi parmi l'indène et le styrène, et de deuxième monomère choisi parmi les dimères du cyclopentanediène tels que le dicyclopentanediène, le méthyldicyclopentanediène, les autres dimères du pentanediène, et leurs mélanges. Ces résines présentent généralement un poids moléculaire allant de 500 à 800 g/mol, telles que par exemple celles commercialisées sous la référence BETAPRENE BR 100 par la société Arizona Chemical Co., NEVILLE LX-685-125 et NEVILLE LX-1000 par la société Neville Chem., PICCODIENE 2215 par la société Hercules, PETRO-REZ 200 par la société Lawter ou RESINALL 760 par la société Resinall Corp. ;
- les résines diènes des dimères de l'isoprène telles que les résines terpèniques issues de la polymérisation d'au moins un monomère choisi parmi $\alpha$-pinène, le $\beta$-pinène, le limonène, et leurs mélanges. Ces résines peuvent présenter un poids moléculaire allant de 300 à 2000 g/mol. De telles résines sont commercialisées par exemple sous la dénomination PICCOLYTE A115 et S 125 par la société Hercules, ZONAREZ 7100 ou ZONATAC 105 LITE par la société ARIZONA Chem ;
- les polyoléfines C6-C20 hydrogénées telles que celles commercialisées sous les dénominations EASTOTAC H-142W, EASTOTAC H-142R, et EASTOTAC H-100W par la société Eastman Chemical Co.

Selon un mode préféré de réalisation, la résine est choisie parmi les résines hydrocarbonées indéniques en particulier les copolymères indène/méthylstyrène/styrène hydrogéné commercialisées sous la dénomination « REGALITE » par la société Eastman Chemical, tels que la REGALITE R 1100, REGALITE R 1090, REGALITE R-7100, REGALITE R1010 HYDROCARBON RESIN, REGALITE R1125 HYDROCARBON RESIN.

La résine tackifiante peut être présente dans la composition selon l'invention en une teneur allant de 0,1 à 45 % en poids, par rapport au poids total de la composition, de préférence allant de 0,5 à 30 % en poids, plus préférentiellement allant de 1 à 15 % en poids.

De façon préférée, le ratio massique résine de poids moléculaire moyen en nombre inférieur ou égal à 10000 g/mol copolymère éthylénique séquencé est compris entre 1/20 et 20/1, de préférence entre 1/10 et 10/1, ou mieux entre 1/5 et 5/1.

Selon ce mode de réalisation selon lequel la composition comprend au moinsune résine telle que décrite précédemment, la composition est de préférence sous forme solide et comprend au moins un corps gras solide, tel qu'une cire ou un corps gras pâteux.

De façon avantageuse, selon ce mode de réalisation, le (ou les) composé pâteux représente, s'il est présent dans la composition, de préférence 0,1 à 80%, mieux 0,5 à 60%, mieux 1 à 30% et mieux encore 1 à 20% en poids par rapport au poids total de la composition.

Selon un mode de réalisation préféré, la composition selon l'invention comprend au moins une cire.

Selon ce mode de réalisation, la composition selon l'invention peut comprendre une teneur en cires allant de 0,1 à 30 % en poids par rapport au poids total de la composition, en particulier elle peut en contenir de 0,5 à 20 %, plus particulièrement de 1 à 15%.

La composition solide selon l'invention présente, à température ambiante (20°C) et à pression atmosphérique (760 mm de Hg), une dureté supérieure à 30 $Nm^{-1}$, dé préférence supérieure à 40 $Nm^{-1}$.

## Polymère filmogène additionnel

La composition peut comprendre, outre le copolymère décrit précédemment, un polymère additionnel tel qu'un polymère filmogène.

Selon la présente invention, on entend par « polymère filmogène », un polymère apte à former à lui seul ou en présence d'un agent auxiliaire de filmification, un dépôt continu sur un support, notamment sur les matières kératiniques.

Parmi les polymères filmogènes utilisables dans la composition de la présente invention, on peut citer les polymères synthétiques, de type radicalaire ou de type polycondensat, les polymères d'origine naturelle et leurs mélanges. Comme polymère filmogène, on peut citer en particulier les polymères acryliques, les polyuréthanes, les polyesters, les polyamides, les polyurées, les polymères cellulosiques comme la nitrocellulose.

Le polymère peut être associé à un ou des agents auxiliaires de filmification. Un tel agent de filmification peut être choisi parmi tous les composés connus de l'homme du métier comme étant susceptibles de remplir la fonction recherchée, et notamment être choisi parmi les agents plastifiants et les agents de coalescence.

## Gélifiant

La composition selon l'invention peut en outre comprendre un gélifiant lipophile.

Il peut en particulier s'agir de gélifiants lipophiles, organiques ou minéraux, polymériques ou moléculaires.

Comme gélifiants lipophiles, on peut citer les argiles, éventuellement modifiées comme les hectorites modifiées, la silice traitée hydrophobe, et leurs mélanges.

## Matière colorante

La composition selon l'invention peut en outre comprendre une matière colorante choisie parmi les colorants hydrosolubles, et les matières colorantes pulvérulentes comme les pigments, les nacres, et les paillettes bien connues de l'homme du métier. Les matières colorantes peuvent être présentes, dans la composition, en une teneur allant de 0,01 % à 50 % en poids, par rapport au poids de la composition, de préférence de 0,01 % à 30 % en poids.

Par pigments, il faut comprendre des particules de toute forme, blanches ou colorées, minérales ou organiques, insolubles dans le milieu physiologique, destinées à colorer la composition.

Par nacres, il faut comprendre des particules de toute forme irisées, notamment produites par certains mollusques dans leur coquille ou bien synthétisées.

Les pigments peuvent être blancs ou colorés, minéraux et/ou organiques. On peut citer, parmi les pigments minéraux, le dioxyde de titane, éventuellement traité en surface, les oxydes de zirconium ou de cérium, ainsi que les oxydes de zinc, de fer (noir, jaune ou rouge) ou de chrome, le violet de manganèse, le bleu outremer, l'hydrate de chrome et le bleu ferrique, les poudres métalliques comme la poudre d'aluminium, la poudre de cuivre.

Parmi les pigments organiques, on peut citer le noir de carbone, les pigments de type D & C, et les laques à base de carmin de cochenille, de baryum, strontium, calcium, aluminium.

On peut également citer les pigments à effet tels les particules comportant un substrat organique ou minéral, naturel ou synthétique, par exemple le verre, les résines acrylique, le polyester, le polyuréthane, le polyéthylène téréphtalate, les céramiques ou les alumines, ledit substrat étant recouvert ou non de substances métalliques comme l'aluminium, l'or, l'argent, le platine, le cuivre, le bronze, ou d'oxydes métalliques comme le dioxyde de titane, l'oxyde de fer, l'oxyde de chrome et leurs mélanges.

Les pigments nacrés peuvent être choisis parmi les pigments nacrés blancs tels que le mica recouvert de titane, ou d'oxychlorure de bismuth, les pigments nacrés colorés tels que le mica titane recouvert avec des oxydes de fer, le mica titane recouvert avec notamment du bleu ferrique ou de l'oxyde de chrome, le mica titane recouvert avec un pigment organique du type précité ainsi que les pigments nacrés à base d'oxychlorure de bismuth. On peut également utiliser les pigments interférentiels, notamment à cristaux liquides ou multicouches.

Les colorants hydrosolubles sont par exemple le jus de betterave, le bleu de méthylène.

## Charges

La composition selon l'invention peut comprendre en outre une ou plusieurs charges, notamment en une teneur allant de 0,01 % à 50 % en poids, par rapport au poids total de la composition, de préférence allant de 0,01 % à 30 % en poids. Par charges, il faut comprendre des particules de toute forme, incolores ou blanches, minérales ou de synthèse, insolubles dans le milieu de la composition quelle que soit la température à laquelle la composition est fabriquée. Ces charges servent notamment à modifier la rhéologie ou la texture de la composition.

Les charges peuvent être minérales ou organiques de toute forme, plaquettaires, sphériques ou oblongues, quelle que soit la forme cristallographique (par exemple feuillet, cubique, hexagonale, orthorombique, etc). On peut citer le talc, le mica, la silice, le kaolin, les poudres de polyamide (Nylon®) (Orgasol® de chez Atochem), de poly-$\beta$-alanine et de polyéthylène, les poudres de polymères de tétrafluoroéthylène (Téflon®), la lauroyl-lysine, l'amidon, le nitrure de bore, les microsphères creuses polymériques telles que celles de chlorure de polyvinylidène/acrylonitrile comme l'Expancel® (Nobel Industrie), de copolymères d'acide acrylique (Polytrap® de la société Dow Corning), le carbonate de calcium précipité, le carbonate et l'hydro-carbonate de magnésium, l'hydroxyapatite, les microsphères de silice creuses (Silica Beads® de Maprecos), les microcapsules de verre ou de céramique, les savons métalliques dérivés d'acides organiques carboxyliques ayant de 8 à 22 atomes de carbone, de préférence de 12 à 18 atomes de carbone, par exemple le stéarate de zinc, de magnésium ou de lithium, le laurate de zinc, le myristate de magnésium.

La composition selon l'invention peut se présenter notamment sous forme de suspension, de dispersion, de solution, de gel, d'émulsion, notamment émulsion huile-dans-eau (H/E) ou eau-dans-huile (E/H), ou multiple (E/H/E ou polyol/H/E ou H/E/H), sous forme de crème, de pâte, de mousse, de dispersion de vésicules notamment de lipides ioniques ou non, de lotion biphase ou multiphase, de spray, de poudre, de pâte, notamment de pâte souple.

De façon préférée, la composition selon l'invention comprend moins de 3%, ou mieux, moins de 1% d'eau en poids par rapport au poids total de la composition. De façon encore préférée la composition est totalement anhydre. Par anhydre, on entend notamment que l'eau n'est de préférence pas ajoutée délibérément dans la composition mais peut être présente à l'état de trace dans les différents composés utilisés dans la composition.

L'homme du métier pourra choisir la forme galénique appropriée, ainsi que sa méthode de préparation, sur la base de ses connaissances générales, en tenant compte, d'une part, de la nature des constituants utilisés, notamment de leur solubilité dans le support, et d'autre part, de l'application envisagée pour la composition.

La composition selon l'invention peut être destinée au soin et/ou au maquillage des matières kératiniques, notamment des lèvres et de la peau, en particulier des lèvres.

La composition selon l'invention peut être sous forme d'un gloss liquide.

La composition selon l'invention peut être sous forme solide, en stick ou coulé en coupelle par exemple.

Les exemples qui suivent illustrent de manière non limitative l'invention.

Les quantités sont exprimées en pourcentage massique.

## Exemples

### Exemple 1 : préparation d'un copolymère de poly (acrylate d'isobornyle / méthacrylate disobornyle / acrylate isobutyle / acide acrylique)

300 g d'isododécane sont introduits dans un réacteur de 1 litre, puis on augmente la température de façon à passer de la température ambiante (25 °C) à 90 °C en 1 heure.

On ajoute ensuite, à 90 °C et en 1 heure, 105 g de méthacrylate d'isobornyle, 105 g d'acrylate d'isobornyle et 1,8 g de 2.5-Bis(2-éthylhexanoylperoxy)-2.5-diméthylhexane (Trigonox® 141 d'Akzo Nobel).

Le mélange est maintenu 1h30 à 90 °C.

On introduit ensuite au mélange précédent, toujours à 90 °C et en 30 minutes, 75 g d'acrylate d'isobutyle, 15 g d'acide acrylique et 1,2 g de 2.5-Bis (2-ethylhexanoylperoxy)-2.5-diméthylhexane.

Le mélange est maintenu 3 heures à 90 °C, puis l'ensemble est refroidi.

On obtient ainsi une solution contenant 50% en matière sèche de copolymère dans 50% d'isododécane comprenant une première séquence ou bloc poly(acrylate d'isobornyle/méthacrylate d'isobornyle) ayant une Tg de 128 °C, une deuxième séquence poly (acrylate d'isobutyle/acide acrylique) ayant une Tg de -9 °C et une séquence intermédiaire qui est un copolymère statistique acrylate d'isobornyle/méthacrylate d'isobornyle/acrylate d'isobutyle/acide acrylique.

La Tg du copolymère est de 74 °C.

Il s'agit de Tg théoriques calculées par la loi de Fox.

**Exemple 2 selon l'invention: Distillation du solvant de synthèse (l'isododécane) par ajout du néopentanoate d'octyldodécyle**

On chauffe la solution obtenue dans l'exemple 1 à 130°C sous un vide de 100 mbar pour évaporer l'isododécane en ajoutant simultanément le néopentanoate d'octyldodécyle. L'intégralité de l'isododécane est substituée par autant de néopentanoate d'octyldodécyle en poids.

L'utilisation du néopentanoate d'octyldodécyle permet d'évaporer la totalité de l'isododécane, celui-ci ne subsistant éventuellement qu'à l'état de traces résiduelles. On obtient ainsi une solution à 50 % de matière sèche en copolymère dans 50% de néopentanoate d'octyldodécyle.

**Exemple 3 comparatif : Distillation du solvant de synthèse (l'isododécane) par ajout de parleam**

On chauffe la solution obtenue dans l'exemple 1 à 130°C sous un vide de 100 mbar pour évaporer l'isododécane en ajoutant simultanément le polyisobutène hydrogéné (Parléam de NOF Corporation).

L'utilisation du parleam ne permet pas d'évaporer la totalité de l'isododécane. On obtient finalement le copolymère en solution dans 37,5% en poids de parleam et 12,5% en poids d'isododécane. Au-delà, de ce seuil, le mélange devient trop visqueux et non manipulable et il n'est plus possible de distiller le reste de l'isododécane.

On obtient ainsi une solution de 50 % de matière sèche en copolymère dans 37,5% en poids de parleam et 12,5% d'isododécane.

**Exemple 4 comparatif : Distillation du solvant de synthèse (l'isododécane) par ajout de Belsil PDM 1000 de Wacker**

On chauffe la solution obtenue dans l'exemple 1 à 130°C sous un vide de 100 mbar pour évaporer l'isododécane en ajoutant simultanément l'huile siliconée TRIMETHYLSILOXYPHENYL DIMETHICONE (Belsil PDM 1000 de Wacker).

L'utilisation de cette huile siliconée ne permet pas d'évaporer la totalité de l'isododécane. On obtient finalement une solution à 50% en poids de copolymère dans 10% en poids de Belsil PDM 1000 et 40% en poids d'isododécane. Au-delà, de ce seuil, le mélange devient trop visqueux et non manipulable et il n'est plus possible de distiller le reste de l'isododécane.

**Exemple 5: Composition de rouge à lèvres en stick**

| | COMPOSES | COMPOSITION 1 SELON L'INVENTION (% en poids) |
|---|---|---|
| Phase A | DIISOSTEARYL MALATE (SCHERCEMOL DISM de LUBRIZOL) | 5,07 |
| | HYDROGENATED POLYISOBUTENE (PARLEAM de NOF CORPORATION) | 11,15 |
| | TRIMETHYL PENTAPHENYL TRISILOXANE (DOW CORNING PH-1555 HRI COSMETIC FLUID de DOW CORNING) | 33,68 |
| | PHENYL TRIMETHICONE (DOW CORNING 556 COSMETIC GRADE FLUID de DOW CORNING) | 5,07 |

(suite)

| | COMPOSES | COMPOSITION 1 SELON L'INVENTION (% en poids) |
|---|---|---|
| Phase B | HYDROGENATED CASTOR OIL / SEBACIC ACID COPOLYMER (CRODABOND CSA de -CRODA) | 11,15 |
| | Poly(méthacrylate d'isobornyle-co-acrylate d'isobornyle-co-acrylate d'isobutyle-co-acide acrylique) à 50 % en matière active dans 50% d'octyldodécyl néopentanoate tel que préparé dans l'exemple 2 ci-dessus (Mexomère PAZ de Chimex) | 8 |
| Phase C | MICROCRYSTALLINE WAX (MICROWAX HW de PARAMELT) | 4,1 |
| | VP/EICOSENE COPOLYMER (ANTARON V 220F OU GANEX V 220F de ISP) | 5 |
| | C20-40 ALKYL STEARATE (KESTER WAX K 82 H de KOSTER KEUNEN) | 6,8 |
| Phase D | IRON OXIDES (SICOMET BRUN ZP 3569 de BASF) | 4,28 |
| | BLUE 1 LAKE CI 42090 | 1,31 |
| | TITANIUM DIOXIDE (TIPAQUE PF-671 de ISHIHARA SANGYO) | 1,79 |
| | RED 28 LAKE de SUNCROMA RED 28 AL LAKE C14-6623 de SUN | 2,6 |
| | Total : | 100 |

La composition de rouge à lèvres est préparée selon le protocole suivant :

Dans un premier temps, les charges et/ou les pigments de la phase D sont broyés à la broyeuse tricylindre dans une partie de la phase huileuse A.

Le reste des ingrédients des phases B et C sont ensuite mélangés à une température de l'ordre de 100°C.
Le broyat pigmentaire et/ou les actifs prédispersés sont alors ajoutés dans le mélange à une température de l'ordre de 100°C.
Enfin, la composition est coulée dans un moule permettant l'obtention de sticks de 12,7 mm de diamètre et le tout est laissé refroidir dans un congélateur environ une heure.
La dureté de la composition est mesurée selon la méthode décrite précédemment et est de 111 $Nm^{-1}$.
La composition appliquée sur les lèvres est agréable à l'application, brillant et présente une bonne tenue de la couleur et de la brillance.
En outre, elle ne nécessite pas d'être conditionnée dans un packaging étanche.

**Exemple 6 : Composition de rouge à lèvres de type gloss liquide**

| NOM | Composition 2 (% massique) |
|---|---|
| Perhydrosqualene végétal raffinée (Nom INCI = squalane) | 10,86 |
| Octyl-2 dodécanol | 15,39 |
| Oxyde de titane rutile traité aluminé/silice/trimethyolpropane | 2,74 |
| RED 7 | 0,54 |
| Lake Blue 1 | 0,16 |
| Lake Yellow 6 | 2,58 |

(suite)

| NOM | Composition 2 (% massique) |
|---|---|
| Oxyde de fer noir | 0,25 |
| Mica - dioxyde de titane - oxyde de fer brun | 2 |
| Polyphenyltrimethylsiloxy dimethylsiloxane (Belsil PDM 1000 de WACKER (viscosité 1000 cPs, PM : 9000) | 20,03 |
| Silice pyrogénée hydrophobe, traitée en surface par diméthylsilane (AEROSIL R 972 de DEGUSSA) | 4,5 |
| Poly(méthacrylate d'isobornyle-co-acrylate d'isobornyle-co-acrylate d'isobutyle-co-acide acrylique) à 50 % en matière active dans 50% d'octyldodécyl néopentanoate tel que préparé dans l'exemple 2 ci-dessus (Mexomère PAZ de Chimex) | 30 |
| Polybutylène (INDOPOL H 100 (PM : 920) d'INEOS) | 10,65 |
| Parfum | 0,3 |
| Total | 100 |

Le mode opératoire pour de la formulation suivante est le suivant :

- Les pigments sont broyés 3 fois à la broyeuse tricylindre dans l'octyldodécanol porté au préalable à 60 °C. Le broyat est laissé à refroidir à température ambiante (25 °C) dans un poêlon double paroi ou un bécher.
- Le copolymère, le squalane, le polybutylène, les nacres et le parfum sont ajoutés au broyat. Le tout est agité à la turbine (type : Rayneri) pour homogénéiser.
- Quand le mélange est homogène, la polyphenyl trimethylsiloxy dimethylsiloxane est ajoutée sous agitation à 800 tours/minute au Rayneri pendant 30 minutes environ.
- Enfin, la sililce pyrogénée est ajoutée en pluie et l'agitation à la turbine est maintenue à 1000 tours/minute pendant 20 minutes.

Cette composition de gloss, appliquée sur les lèvres en un seul geste, présente des propriétés confort et de brillance satisfaisantes.
La composition présente en outre une bonne tenue de la couleur et de la brillance.
Elle ne nécessite pas d'être conditionnée dans un packaging étanche.

**Exemples 7 et 8 : Rouges à lèvres en stick**

On a préparé une composition 4 de rouge à lèvres selon l'invention et une composition 3 comparative ne faisant pas partie de l'invention, comprenant les ingrédients suivants (quantités en pourcentage massique) :

| | **Matières premières (Nom INCI US)** | **Composition 3 comparative (hors invention)** | **Composition 4 selon l'invention** |
|---|---|---|---|
| Phase A | NEOPENTANOATE D'OCTYLDODECYL | 17,31 | 7,42 |
| | POLYISOBUTENE HYDROGENE (PARLEAM de chez NOF Corporation) | 6,53 | 6,53 |
| | VinylPyrrolidone/HEXADECENE COPOLYMER (Antaron V216 de chez ISP) | 8,21 | 8,21 |
| Phase B | ISOHEXADECANE | 18,30 | 18,30 |

(suite)

| | Matières premières (Nom INCI US) | Composition 3 comparative (hors invention) | Composition 4 selon l'invention |
|---|---|---|---|
| | COPOLYMERE STYRENE/METHYL STYRENE/INDENE HYDROGENE (REGALITE R1100 de chez EASTMAN CHEMICAL) | 7,91 | 7,91 |
| | Poly(méthacrylate d'isobornyle-co-acrylate d'isobornyle-co-acrylate d'isobutyle-co-acide acrylique) à 50 % en matière active dans 50% d'octyldodécyl néopentanoateselon l'exemple 2 ci-dessus | - | 9,89 |
| Phase C | COPOLYMERE VinylPyrrolidone /EICOSENE (Antaron V220F de chez ISP) | 1,98 | 1,98 |
| | CIRE DE POLYETHYLENE (Performalene 500-L de New Phase Technologies) | 10,88 | 10,88 |
| Phase D | Oxydes de fer | 3,39 | 3,39 |
| | Colorant bleu | 1,04 | 1,04 |
| | Dioxyde de titane | 1,41 | 1,41 |
| | colorant rouge | 2,06 | 2,06 |
| Phase E | TRIMETHYLSILOXYPHENYL DIMETHICONE (BELSIL 1000 de chez Wacker) | 20,97 | 20,97 |
| | Total : | 100 | 100 |

Les compositions 3 et 4 sont obtenues selon le protocole suivant :

Dans un premier temps, les charges et les pigments de la phase D sont broyés à la broyeuse tri-cylindre dans une partie de la phase huileuse.

Le reste des ingrédients liposolubles sont ensuite mélangés à une température de l'ordre de 100 °C.
Le broyat ou les actifs pré-dispersés sont alors ajoutés dans la phase huileuse.
Enfin, la composition est coulée dans un moule permettant l'obtention de sticks de 12,7 mm de diamètre et le tout est laissé refroidir dans un congélateur environ une heure.

Lors de l'application sur les lèvres de chacune des deux compositions 3 et 4, le rouge à lèvres de composition 4 selon l'invention, par comparaison avec la composition comparative 3 ne contenant pas de copolymère séquencé, est plus onctueux à l'application et le dépôt formé est plus épais et plus confortable.

De plus, 1heure après application, on observe que le dépôt sur les lèvres réalisé avec la composition 4 reste plus épais et plus confortable et présente une meilleure tenue de la brillance et de la couleur que celui réalisé avec la composition 3.

## Revendications

1. Composition cosmétique pour le maquillage et/ou le soin des matières kératiniques comprenant, dans un milieu physiologiquement acceptable, au moins :

    a) un copolymère éthylénique séquencé (également appelé polymère éthylénique séquencé), contenant au moins une première séquence ayant une température de transition vitreuse (Tg) supérieure ou égale à 40°C et étant issue en totalité ou en partie de un ou plusieurs premiers monomères, qui sont tels que l'homopolymère préparé à partir de ces monomères a une température de transition vitreuse supérieure ou égale à 40°C, ladite première séquence étant obtenue à partir :

- d'au moins un monomère méthacrylate de formule $CH_2 = C(CH_3)\text{-}COOR'_2$ dans laquelle $R'_2$ représente un groupe cycloalkyle $C_4$ à $C_{12}$, et
- d'au moins un monomère acrylate de formule $CH_2 = CH\text{-}COOR_2$

dans laquelle $R_2$ représente un groupe cycloalkyle en $C_4$ à $C_{12}$,
et au moins une deuxième séquence ayant une température de transition vitreuse inférieure ou égale à 20°C et étant issue en totalité ou en partie de un ou plusieurs deuxièmes monomères, qui sont tels que l'homopolymère préparé à partir de ces monomères a une température de transition vitreuse inférieure ou égale à 20°C, et d'un monomère additionnel choisi parmi l'acide acrylique ;
ladite première séquence et ladite deuxième séquence étant reliées entre elles par un segment intermédiaire statistique comprenant au moins un desdits premiers monomères constitutifs de la première séquence et au moins un desdits deuxièmes monomères constitutifs de la deuxième séquence, et ledit copolymère séquencé ayant un indice de polydispersité I supérieur à 2, et au moins
b) une huile ester hydrocarbonée non volatile comprenant au moins 16 atomes de carbone et ayant une masse molaire inférieure à 650 g/moles;

ladite composition comprenant moins de 5 % en poids d'huile volatile dont le point éclair est inférieur ou égal à 80°C, par rapport au poids total de la composition.

2. Composition selon la revendication précédente, **caractérisée en ce qu'**elle ne comprend pas d'huile volatile dont le point éclair est inférieur ou égal à 80°C.

3. Composition selon l'une quelconque des revendications précédentes, **caractérisée en ce que**
le ou lesdits deuxièmes monomères, qui sont tels que l'homopolymère préparé à partir de ces monomères a une températures de transition vitreuse inférieure ou égale à 20°C, sont choisis parmi :

- les acrylates de formule $CH_2 = CHCOOR_3$,
$R_3$ représentant un groupe alkyle non substitué en $C_1$ à $C_{12}$, linéaire ou ramifié, à l'exception du groupe tertiobutyle, dans lequel se trouve(nt) éventuellement intercalé(s) un ou plusieurs hétéroatomes choisis parmi O, N, S,
- les méthacrylates de formule $CH_2 = C(CH_3)\text{-}COOR_4$,
$R_4$ représentant un groupe alkyle non substitué en $C_6$ à $C_{12}$ linéaire ou ramifié, dans lequel se trouve(nt) éventuellement intercalé(s) un ou plusieurs hétéroatomes choisis parmi 0, N et S;
- les esters de vinyle de formule $R_5\text{-CO-O-CH} = CH_2$
où $R_5$ représente un groupe alkyle en $C_4$ à $C_{12}$ linéaire ou ramifié ;
- les éthers d'alcool vinylique et d'alcool en $C_4$ à $C_{12}$,
- les N-alkyl en $C_4$ à $C_{12}$ acrylamides, tels que le N-octylacrylamide,
- et leurs mélanges.

4. Composition cosmétique selon l'une quelconque des revendications précédentes, **caractérisée en ce que** le copolymère éthylénique séquencé est un copolymère comprenant au moins un monomère acrylate de formule $CH_2 = CH\text{-}COOR_2$ dans laquelle $R_2$ représente un groupe cycloalkyle $C_8$ à $C_{12}$ et au moins un monomère méthacrylate de formule $CH_2 = C(CH_3)\text{-}COOR'_2$ dans laquelle $R'_2$ représente un groupe cycloalkyle $C_8$ à $C_{12}$, au moins un deuxième monomère acrylate de formule $CH_2 = CHCOOR_3$, dans laquelle $R_3$ représente un groupe alkyle non substitué en $C_1$ à $C_{12}$, linéaire ou ramifié, à l'exception du groupe tertiobutyle, et au moins un monomère acide acrylique.

5. Composition selon l'une quelconques des revendications 1 à 4, **caractérisée en ce que** $R_2$ et $R'_2$ représentent indépendamment ou simultanément un groupe isobornyle.

6. Composition selon l'une quelconques des revendications précédentes, **caractérisée en ce que** ledit copolymère est obtenu à partir d'au moins un monomère méthacrylate d'isobornyle, au moins un monomère acrylate d'isobornyle, au moins un monomère acrylate d'isobutyle et au moins un monomère d'acide acrylique.

7. Composition selon l'une quelconque des revendications précédentes, **caractérisée en ce que** ledit copolymère comprend de 50 à 80 % en poids de méthacrylate/acrylate d'isobornyle, de 10 à 30 % en poids d'acrylate d'isobutyle et de 2 à 10 % en poids d'acide acrylique.

8. Composition selon l'une quelconque des revendications précédentes, **caractérisée en ce qu'**elle comprend de 0,5

à 40 % en poids de matière active de copolymère séquencé, et avantageusement de 1 à 40 % en poids, notamment de 2 à 30 % en poids, voire de 2 à 20 % en poids de matière active de copolymère par rapport au poids total de la composition.

**9.** Composition selon l'une quelconque des revendications précédentes, **caractérisée en ce que** ladite huile hydrocarbonée non volatile comprenant au moins 16 atomes de carbone et ayant une masse molaire inférieure à 650 g/moles est l'octyldodécyl néopentanoate.

**10.** Composition cosmétique selon l'une quelconque des revendications précédentes, **caractérisée en ce que** le ratio massique entre ledit copolymère séquencé et l'huile ester hydrocarbonée non volatile comprenant au moins 16 atomes de carbone et ayant une masse molaire inférieure à 650 g/moles, est inférieur à 1.

**11.** Composition cosmétique selon l'une quelconque des revendications précédentes, **caractérisée en ce qu'**elle comprend moins de 3 % d'eau, ou mieux moins de 1 % ou encore est totalement anhydre.

**12.** Composition cosmétique selon l'une quelconque des revendications précédentes, **caractérisée en ce qu'**elle comprend en outre au moins une huile volatile dont le point éclair est supérieur à 80°C.

**13.** Composition cosmétique selon l'une quelconque des revendications précédentes, **caractérisée en ce qu'**elle comprend au moins une huile non volatile additionnelle, et/ou une charge et/ou une matière colorante.

**14.** Composition cosmétique selon l'une quelconque des revendications précédentes, **caractérisée en ce qu'**elle comprend au moins un corps gras solide choisi parmi les cires et/ou les corps gras pâteux, la composition étant de préférence sous forme solide.

**15.** Composition cosmétique selon la revendication précédente, **caractérisée en ce qu'**elle est sous forme solide et **en ce qu'**elle comprend au moins une résine de poids moléculaire moyen en nombre inférieur ou égal à 10000 g/mol choisie parmi les résines hydrocarbonées indéniques, de préférence hydrogénées, et leurs mélanges.

**16.** Composition selon la revendication précédente, **caractérisée en ce que** la résine hydrocarbonée indénique est hydrogénée.

**17.** Composition selon l'une quelconque des revendications précédentes, **caractérisée en ce que** la résine est une résine indénique choisie parmi les copolymères indène/méthylstyrène/styrène hydrogénés.

**18.** Composition selon l'une quelconque des revendications précédentes, **caractérisée en ce que** la résine est présente en une teneur allant de 0,1 à 30 % en poids, par rapport au poids total de la composition, de préférence allant de 0,3 à 20 % en poids, plus préférentiellement allant de 0,5 à 15 % en poids.

**19.** Procédé cosmétique de maquillage des matières kératiniques comprenant l'application sur lesdites matières kératiniques, et notamment les lèvres ou la peau, d'une composition telle que définie dans l'une quelconque des revendications précédentes.

## Patentansprüche

**1.** Kosmetikzusammensetzung zum Schminken und/oder zur Pflege von Keratinsubstanzen, die in einem physiologisch annehmbaren Medium mindestens Folgendes umfasst :

a) ein Ethylen-Block-Copolymer (auch als Ethylen-Blockpolymer bezeichnet) mit mindestens einem ersten Block, der eine Glasübergangstemperatur (Tg) von mehr als oder gleich 40°C hat und vollständig oder zum Teil von einem oder mehreren ersten Monomeren herrührt, die derart sind, dass ein ausgehend von diesen Monomeren hergestelltes Homopolymer eine Glasübergangstemperatur von mehr als oder gleich 40°C hat, wobei der erste Block aus den Folgenden erhalten wird:

- mindestens einem Methacrylatmonomer der Formel $CH_2=C(CH_3)-COOR'_2$, wobei $R'_2$ für eine $C_4$- bis $C_{12}$-Cycloalkylgruppe steht, und
- mindestens einem Acrylatmonomer der Formel $CH_2=CH-COOR_2$, wobei $R_2$ für eine $C_4$- bis $C_{12}$-Cycloal-

kylgruppe steht,

und mindestens einem zweiten Block, der eine Glasübergangstemperatur von weniger als oder gleich 20°C hat und vollständig oder zum Teil von einem oder mehreren zweiten Monomeren herrührt, die derart sind, dass ein aus diesen Monomeren hergestelltes Homopolymer eine Glasübergangstemperatur von weniger als oder gleich 20°C hat, und einem aus Acrylsäure ausgewählten zusätzlichen Monomer;
wobei der erste Block und der zweite Block miteinander durch ein statistisches Zwischensegment verbunden sind, das mindestens eines der ersten Monomere, die den ersten Block bilden, und mindestens eines der zweiten Monomere, die den zweiten Block bilden, umfasst, und wobei das Block-Copolymer einen Polydispersitätsindex I von mehr als 2 hat, und mindestens
b) ein nichtflüchtiges Kohlenwasserstoffesteröl, das mindestens 16 Kohlenstoffatome umfasst und eine Molekülmasse unter 650 g/mol hat;

wobei die Zusammensetzung weniger als 5 Gew.-% flüchtiges Öl mit einem Flammpunkt von weniger als oder gleich 80°C, bezogen auf das Gesamtgewicht der Zusammensetzung, umfasst.

2. Zusammensetzung nach dem vorhergehenden Anspruch, **dadurch gekennzeichnet, dass** sie kein flüchtiges Öl mit einen Flammpunkt von weniger als oder gleich 80°C umfasst.

3. Zusammensetzung nach einem der vorhergehenden Ansprüche, **dadurch gekennzeichnet, dass** das oder die zweite(n) Monomer(e), die derart sind, dass das ausgehend von diesen Monomeren hergestellte Homopolymer eine Glasübergangstemperatur von weniger als oder gleich 20°C hat, aus Folgenden ausgewählt ist/sind:

- Acrylaten der Formel $CH_2=CHCOOR_3$,
wobei $R_3$ für eine unsubstituierte, gerade oder verzweigte $C_1$- bis $C_{12}$-Alkylgruppe mit Ausnahme der tert-Butylgruppe steht, in der sich gegebenenfalls ein oder mehrere aus O, N, S ausgewählte interkalierte Heteroatome befinden,
- Methacrylaten der Formel $CH_2=C(CH_3)-COOR_4$,
wobei $R_4$ für eine unsubstituierte, gerade oder verzweigte $C_6$- bis $C_{12}$-Alkylgruppe steht, in der sich gegebenenfalls ein oder mehrere aus O, N und S ausgewählte interkalierte Heteroatome befinden,
- Vinylestern der Formel $R_5$-CO-O-CH=$CH_2$,
wobei $R_5$ für eine gerade oder verzweigte $C_4$- bis $C_{12}$-Alkylgruppe steht;
- Vinylalkohol- und $C_4$- bis $C_{12}$-Alkoholethern,
- N-($C_4$- bis $C_{12}$-Alkyl)acrylamiden, wie N-Octylacrylamid,

und deren Gemischen.

4. Kosmetikzusammensetzung nach einem der vorhergehenden Ansprüche, **dadurch gekennzeichnet, dass** das Ethylen-Block-Copolymer ein Copolymer ist, das mindestens ein Acrylatmonomer der Formel $CH_2=CH-COOR_2$, wobei $R_2$ für eine $C_8$- bis $C_{12}$-Cycloalkylgruppe steht, und mindestens ein Methacrylatmonomer der Formel $CH_2=C(CH_3)$ - $COOR'_2$, wobei $R'_2$ für eine $C_8$- bis $C_{12}$-Cycloalkylgruppe steht, mindestens ein zweites Acrylatmonomer der Formel $CH_2=CHCOOR_3$, wobei $R_3$ für eine unsubstituierte gerade oder verzweigte $C_1$- bis $C_{12}$-Alkylgruppe mit Ausnahme der tert-Butylgruppe steht, und mindestens ein Acrylsäuremonomer umfasst.

5. Zusammensetzung nach einem der Ansprüche 1 bis 4, **dadurch gekennzeichnet, dass** $R_2$ und $R'_2$ unabhängig voneinander oder gleichzeitig eine Isobornylgruppe darstellen.

6. Zusammensetzung nach einem der vorhergehenden Ansprüche, **dadurch gekennzeichnet, dass** das Copolymer ausgehend von mindestens einem Isobornylmethacrylatmonomer, mindestens einem Isobornylacrylatmonomer, mindestens einem Isobutylacrylatmonomer und mindestens einem Acrylsäuremonomer erhalten wird.

7. Zusammensetzung nach einem der vorhergehenden Ansprüche, **dadurch gekennzeichnet, dass** das Copolymer 50 bis 80 Gew.-% Isobornylmethacrylat/-acrylat, 10 bis 30 Gew.-% Isobutylacrylat und 2 bis 10 Gew.-% Acrylsäure umfasst.

8. Zusammensetzung nach einem der vorhergehenden Ansprüche, **dadurch gekennzeichnet, dass** sie 0,5 bis 40 Gew.-% aktive Block-Copolymer-Substanz und vorteilhafterweise 1 bis 40 Gew.-%, insbesondere 2 bis 30 Gew.-% oder sogar 2 bis 20 Gew.-% aktive Block-Copolymer-Substanz, bezogen auf das Gesamtgewicht der Zusammensetzung, umfasst.

**9.** Zusammensetzung nach einem der vorhergehenden Ansprüche, **dadurch gekennzeichnet, dass** das nichtflüchtige Kohlenwasserstofföl mindestens 16 Kohlenstoffatome umfasst und eine Molekülmasse von weniger als 650 g/mol hat und Octyldodecylneopentanoat ist.

**10.** Kosmetikzusammensetzung nach einem der vorhergehenden Ansprüche, **dadurch gekennzeichnet, dass** das Massenverhältnis zwischen dem Block-Copolymer und dem nichtflüchtigen Kohlenwasserstoffesteröl, das mindestens 16 Kohlenstoffatome umfasst und eine Mölekülmasse von weniger als 650 g/mol hat, kleiner als 1 ist.

**11.** Kosmetikzusammensetzung nach einem der vorhergehenden Ansprüche, **dadurch gekennzeichnet, dass** sie weniger als 3% Wasser oder besser weniger als 1% umfasst oder auch vollständig wasserfrei ist.

**12.** Kosmetikzusammensetzung nach einem der vorhergehenden Ansprüche, **dadurch gekennzeichnet, dass** sie außerdem mindestens ein flüchtiges Ö1 mit einem Flammpunkt von mehr als 80°C umfasst.

**13.** Kosmetikzusammensetzung nach einem der vorhergehenden Ansprüche, **dadurch gekennzeichnet, dass** sie mindestens ein zusätzliches nichtflüchtiges Öl und/oder einen Füllstoff und/oder eine Färbesubstanz umfasst.

**14.** Kosmetikzusammensetzung nach einem der vorhergehenden Ansprüche, **dadurch gekennzeichnet, dass** sie mindestens eine aus Wachsen und/oder pastösen Fettsubstanzen ausgewählte feste Fettsubstanz umfasst, wobei die Zusammensetzung vorzugsweise in fester Form vorliegt.

**15.** Kosmetikzusammensetzung nach dem vorhergehenden Anspruch, **dadurch gekennzeichnet, dass** sie in fester Form vorliegt, und dadurch, dass sie mindestens ein Harz mit einer zahlengemittelten Molekülmasse von weniger als oder gleich 10000 g/mol umfasst, das aus vorzugsweise hydrierten Inden-Kohlenwasserstoffharzen und deren Gemischen ausgewählt ist.

**16.** Zusammensetzung nach dem vorhergehenden Anspruch, **dadurch gekennzeichnet, dass** das Inden-Kohlenwasserstoffharz hydriert ist.

**17.** Zusammensetzung nach einem der vorhergehenden Ansprüche, **dadurch gekennzeichnet, dass** es sich bei dem Harz um ein aus hydrierten Inden/Methylstyrol/Styrol-Copolymeren ausgewähltes Inden-Harz handelt.

**18.** Zusammensetzung nach einem der vorhergehenden Ansprüche, **dadurch gekennzeichnet, dass** das Harz in einem Gehalt von 0,1 bis 30 Gew.-%, bezogen auf das Gesamtgewicht der Zusammensetzung, vorzugsweise von 0,3 bis 20 Gew.-%, stärker bevorzugt von 0,5 bis 15 Gew.-% vorliegt.

**19.** Kosmetikverfahren zum Schminken von Keratinsubstanzen, das das Auftragen einer Zusammensetzung nach einem der vorhergehenden Ansprüche auf die Keratinsubstanzen, insbesondere auf die Lippen oder die Haut, umfasst.

## Claims

**1.** Cosmetic composition for making up and/or caring for keratinous substances comprising, in a physiologically acceptable medium, at least:

a) one ethylenic block copolymer (also referred to as ethylenic block polymer) comprising at least one first block having a glass transition temperature (Tg) of greater than or equal to 40°C and resulting, in all or in part, from one or more first monomers which are such that the homopolymer prepared from these monomers has a glass transition temperature of greater than or equal to 40°C, the said first block being obtained from:

- at least one methacrylate monomer of formula $CH_2=C(CH_3)-COOR'_2$ in which $R'_2$ represents a $C_4$ to $C_{12}$ cycloalkyl group, and
- at least one acrylate monomer of formula $CH_2=CH-COOR_2$ in which $R_2$ represents a $C_4$ to $C_{12}$ cycloalkyl group,

and at least one second block having a glass transition temperature of less than or equal to 20°C and resulting,

in all or in part, from one or more second monomers which are such that the homopolymer prepared from these monomers has a glass transition temperature of less than or equal to 20°C, and from an additional monomer chosen from acrylic acid;

the said first block and the said second block being connected to one another via a random intermediate segment comprising at least one of the said first constituent monomers of the first block and at least one of the said second constituent monomers of the second block and the said block copolymer having a polydispersity index I of greater than 2, and at least

b) one nonvolatile hydrocarbon ester oil comprising at least 16 carbon atoms and having a molar mass of less than 650 g/mol;

the said composition comprising less than 5% by weight of volatile oil having a flash point of less than or equal to 80°C, with respect to the total weight of the composition.

2. Composition according to the preceding claims, **characterized in that** it does not comprise volatile oil having a flash point of less than or equal to 80°C.

3. Composition according to either one of the preceding claims, **characterized in that** the said second monomer or monomers, which are such that the homopolymer prepared from these monomers has a glass transition temperature of less than or equal to 20°C, are chosen from;

- acrylates of formula $CH_2=CHCOOR_3$,
$R_3$ representing an unsubstituted linear or branched $C_1$ to $C_{12}$ alkyl group, with the exception of the tert-butyl group, in which is(are) optionally inserted one or more heteroatoms chosen from 0, N and S,
- methacrylates of formula $CH_2=C(CH_3)-COOR_4$,
$R_4$ representing an unsubstituted linear or branched $C_6$ to $C_{12}$ alkyl group in which is(are) optionally inserted one or more heteroatoms chosen from 0, N and S;
- vinyl esters of formula $R_5-CO-O-CH=CH_2$,
where $R_5$ represents a linear or branched $C_4$ to $C_{12}$ alkyl group;
- $C_4$ to $C_{12}$ alkyl vinyl ethers,
- N-($C_4$ to $C_{12}$ alkyl)acrylamides, such as N-octylacrylamide,
- and their mixtures.

4. Cosmetic composition according to any one of the preceding claims, **characterized in that** the ethylenic block copolymer is a copolymer comprising at least one acrylate monomer of formula $CH_2=CH-COOR_2$ in which $R_2$ represents a $C_8$ to $C_{12}$ cycloalkyl group and at least one methacrylate monomer of formula $CH_2=C(CH_3)-COOR'_2$ in which $R'_2$ represents a $C_8$ to $C_{12}$ cycloalkyl group, at least one second acrylate monomer of formula $CH_2=CHCOOR_3$ in which $R_3$ represents an unsubstituted linear or branched $C_1$ to $C_{12}$ alkyl group, with the exception of the tert-butyl group, and at least one acrylic acid monomer.

5. Composition according to any one of Claims 1 to 4, **characterized in that** $R_2$ and $R'_2$ represent, independently or simultaneously, an isobornyl group.

6. Composition according to any one of the preceding claims, **characterized in that** the said copolymer is obtained from at least one isobornyl methacrylate monomer, at least one isobornyl acrylate monomer, at least one isobutyl acrylate monomer and at least one acrylic acid monomer.

7. Composition according to any one of the preceding claims, **characterized in that** the said copolymer comprises from 50 to 80% by weight of isobornyl methacrylate/acrylate, from 10 to 30% by weight of isobutyl acrylate and from 2 to 10% by weight of acrylic acid.

8. Composition according to any one of the preceding claims, **characterized in that** it comprises from 0.5 to 40% by weight of block copolymer active material and advantageously from 1 to 40% by weight, in particular from 2 to 30% by weight, indeed even from 2 to 20% by weight, of copolymer active material, with respect to the total weight of the composition.

9. Composition according to any one of the preceding claims, **characterized in that** the said nonvolatile hydrocarbon oil comprising at least 16 carbon atoms and having a molar mass of less than 650 g/mol is octyldodecyl neopentanoate.

**10.** Cosmetic composition according to any one of the preceding claims, **characterized in that** the ratio by weight of the said block copolymer to the nonvolatile hydrocarbon ester oil comprising at least 16 carbon atoms and having a molar mass of less than 650 g/mol is less than 1.

**11.** Cosmetic composition according to any one of the preceding claims, **characterized in that** it comprises less than 3% of water or better still less than 1% or even is entirely anhydrous.

**12.** Cosmetic composition according to any one of the preceding claims, **characterized in that** it additionally comprises at least one volatile oil having a flash point of greater than 80°C.

**13.** Cosmetic composition according to any one of the preceding claims, **characterized in that** it comprises at least one additional nonvolatile oil and/or one filler and/or one colouring material.

**14.** Cosmetic composition according to any one of the preceding claims, **characterized in that** it comprises at least one solid fatty substance chosen from waxes and/or pasty fatty substances, the composition preferably being in the solid form.

**15.** Cosmetic composition according to the preceding claim, **characterized in that** it is in the solid form and **in that** it comprises at least one resin with a number-average molecular weight of less than or equal to 10 000 g/mol chosen from indene hydrocarbon resins, which are preferably hydrogenated, and their mixtures.

**16.** Composition according to the preceding claim, **characterized in that** the indene hydrocarbon resin is hydrogenated.

**17.** Composition according to any one of the preceding claims, **characterized in that** the resin is an indene resin chosen from hydrogenated indene/methylstyrene/styrene copolymers.

**18.** Composition according to any one of the preceding claims, **characterized in that** the resin is present in a content ranging from 0.1 to 30% by weight, with respect to the total weight of the composition, preferably ranging from 0.3 to 20% by weight, more preferably ranging from 0.5 to 15% by weight.

**19.** Cosmetic method for making up keratinous substances comprising the application, to the said keratinous substances and in particular the lips or the skin, of a composition as defined in any one of the preceding claims.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- EP 1411069 A
- EP 1882709 A
- EP 1884229 A
- EP 1184028 A
- EP 0955039 A
- FR 2792190 A


### Littérature non-brevet citée dans la description


- Polymer Handbook. John Wiley, 1989
- Handbook of Pressure Sensitive Adhesive. 1989, 609-619